# EUROPEAN PATENT SPECIFICATION

(11) **EP 2 344 539 B1**
(45) Date of publication and mention of the grant of the patent: **11.02.2015**
(21) Application number: 09764710.1
(22) Date of filing: 06.11.2009
(51) Int. Cl.: C07K 16/30, C07K 16/28, C07K 16/46, A61P 35/02

(54) **TREATMENT OF PEDIATRIC ACUTE LYMPHOBLASTIC LEUKEMIA**
BEHANDLUNG VON PÄDIATRISCHER AKUTER LYMPHOBLASTENLEUKÄMIE
TRAITEMENT DE LA LEUCÉMIE LYMPHOBLASTIQUE AIGUË DE L ENFANT ET DE L ADOLESCENT

(30) Priority: 07.11.2008 US 112323 P; 02.06.2009 US 183291 P; 29.06.2009 US 221269 P
(43) Date of publication of application: 20.07.2011
(73) Proprietor: Amgen Research (Munich) GmbH, 81477 München (DE)
(72) Inventor: ZUGMAIER, Gerhard, 80336 München (DE)
(74) Representative: Schiweck, Weinzierl & Koch
(86) International application number: PCT/EP2009/007969
(87) International publication number: WO 2010/052013

(56) References cited:
- WO-A1-98/08875
- WO-A1-2007/068354
- BARGOU RALF ET AL: "Tumor regression in cancer patients by very low doses of a T cell-engaging antibody." SCIENCE (NEW YORK, N.Y.) 15 AUG 2008, vol. 321, no. 5891, 15 August 2008 (2008-08-15), pages 974-977, XP002572417 ISSN: 1095-9203
- ANDERSON P M ET AL: "G19.4(alpha CD3) x B43(alpha CD19) monoclonal antibody heteroconjugate triggers CD19 antigen-specific lysis of t(4;11) acute lymphoblastic leukemia cells by activated CD3 antigen-positive cytotoxic T cells." BLOOD 1 DEC 1992, vol. 80, no. 11, 1 December 1992 (1992-12-01), pages 2826-2834, XP002572418 ISSN: 0006-4971
- LÖFFLER A ET AL: "A recombinant bispecific single-chain antibody, CD19 x CD3, induces rapid and high lymphoma-directed cytotoxicity by unstimulated T lymphocytes." BLOOD 15 MAR 2000, vol. 95, no. 6, 15 March 2000 (2000-03-15) , pages 2098-2103, XP002572419 ISSN: 0006-4971
- Anomynous: "Phase II Study of the BiTE® Blinatumomab (MT103) in Patients With Minimal Residual Disease of B-Precursor Acute ALL" 11 August 2008 (2008-08-11), 11 August 2008 (2008-08-11), XP002572438 Retrieved from the Internet: URL:http://clinicaltrials.gov/archive/NCT0 0560794/2008_08_11> [retrieved on 2010-03-10]
- PUI CHING-HON ET AL: "Acute lymphoblastic leukaemia." LANCET 22 MAR 2008, vol. 371, no. 9617, 22 March 2008 (2008-03-22), pages 1030-1043, XP002574385 ISSN: 1474-547X
- HANDGRETINGER R ET AL: "Complete remission after blinattumomab-induced donor T-cell activation in three pediatric patients with post-transplant relapsed acutre lymphoblastic leukemia", LEUKEMIA, vol. 25, 2011, pages 181-184,

### SEQUENCE LISTING

<110> Micromet AG
<120> Novel treatment of pediatric acute lymphoblastic leukemia
<130> R2201 PCT S3
<150> 61/112,323
   <151> 2008-11-07
<150> 61/221,269
   <151> 2009-06-29
<150> 61/183,291
   <151> 2009-06-02
<160> 22
<170> PatentIn version 3.3
<210> 1
   <211> 498
   <212> PRT
   <213> Artificial Sequence
<220>
   <221> source
   <223> /note="Description of artificial sequence: CD19xCD3 bispecific single chain antibody"
<400> 1
   Asp Ile Gln Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ser Gln Val
   115 120 125
   Gln Leu Gln Gln Ser Gly Ala Glu Leu Val Arg Pro Gly Ser Ser Val
   130 135 140
   Lys Ile Ser Cys Lys Ala Ser Gly Tyr Ala Phe Ser Ser Tyr Trp Met
   145 150 155 160
   Asn Trp Val Lys Gln Arg Pro Gly Gln Gly Leu Glu Trp Ile Gly Gln
   165 170 175
   Ile Trp Pro Gly Asp Gly Asp Thr Asn Tyr Asn Gly Lys Phe Lys Gly
   180 185 190
   Lys Ala Thr Leu Thr Ala Asp Glu Ser Ser Ser Thr Ala Tyr Met Gln
   195 200 205
   Leu Ser Ser Leu Ala Ser Glu Asp Ser Ala Val Tyr Phe Cys Ala Arg
   210 215 220
   Arg Glu Thr Thr Thr Val Gly Arg Tyr Tyr Tyr Ala Met Asp Tyr Trp
   225 230 235 240
   Gly Gln Gly Thr Thr Val Thr Val Ser Ser Gly Gly Gly Gly Ser Asp
   245 250 255
   Ile Lys Leu Gln Gln Ser Gly Ala Glu Leu Ala Arg Pro Gly Ala Ser
   260 265 270
   Val Lys Met Ser Cys Lys Thr Ser Gly Tyr Thr Phe Thr Arg Tyr Thr
   275 280 285
   Met His Trp Val Lys Gln Arg Pro Gly Gln Gly Leu Glu Trp Ile Gly
   290 295 300
   Tyr Ile Asn Pro Ser Arg Gly Tyr Thr Asn Tyr Asn Gln Lys Phe Lys
   305 310 315 320
   Asp Lys Ala Thr Leu Thr Thr Asp Lys Ser Ser Ser Thr Ala Tyr Met
   325 330 335
   Gln Leu Ser Ser Leu Thr Ser Glu Asp Ser Ala Val Tyr Tyr Cys Ala
   340 345 350
   Arg Tyr Tyr Asp Asp His Tyr Cys Leu Asp Tyr Trp Gly Gln Gly Thr
       355                 360                 365
   Thr Leu Thr Val Ser Ser Val Glu Gly Gly Ser Gly Gly Ser Gly Gly
       370                 375                 380
   Ser Gly Gly Ser Gly Gly Val Asp Asp Ile Gln Leu Thr Gln Ser Pro
   385                 390                 395                 400
   Ala Ile Met Ser Ala Ser Pro Gly Glu Lys Val Thr Met Thr Cys Arg
                   405                 410                 415
   Ala Ser Ser Ser Val Ser Tyr Met Asn Trp Tyr Gln Gln Lys Ser Gly
               420                 425                 430
   Thr Ser Pro Lys Arg Trp Ile Tyr Asp Thr Ser Lys Val Ala Ser Gly
           435                 440                 445
   Val Pro Tyr Arg Phe Ser Gly Ser Gly Ser Gly Thr Ser Tyr Ser Leu
       450                 455                 460
   Thr Ile Ser Ser Met Glu Ala Glu Asp Ala Ala Thr Tyr Tyr Cys Gln
   465                 470                 475                 480
   Gln Trp Ser Ser Asn Pro Leu Thr Phe Gly Ala Gly Thr Lys Leu Glu
                   485                 490                 495
   Leu Lys
<210> 2
   <211> 1494
   <212> DNA
   <213> Artificial Sequence
<220>
   <221> source
   <223> /note="Description of artificial sequence: CD19xCD3 bispecific single chain antibody"
<400> 2
   gatatccagc tgacccagtc tccagcttct ttggctgtgt ctctagggca gagggccacc     60
   atctcctgca aggccagcca aagtgttgat tatgatggtg atagttattt gaactggtac    120
   caacagattc caggacagcc acccaaactc ctcatctatg atgcatccaa tctagtttct    180
   gggatcccac ccaggtttag tggcagtggg tctgggacag acttcaccct caacatccat    240
   cctgtggaga aggtggatgc tgcaacctat cactgtcagc aaagtactga ggatccgtgg     300
   acgttcggtg gagggaccaa gctcgagatc aaaggtggtg gtggttctgg cggcggcggc     360
   tccggtggtg gtggttctca ggtgcagctg cagcagtctg gggctgagct ggtgaggcct     420
   gggtcctcag tgaagatttc ctgcaaggct tctggctatg cattcagtag ctactggatg     480
   aactgggtga agcagaggcc tggacagggt cttgagtgga ttggacagat ttggcctgga     540
   gatggtgata ctaactacaa tggaaagttc aagggtaaag ccactctgac tgcagacgaa     600
   tcctccagca cagcctacat gcaactcagc agcctagcat ctgaggactc tgcggtctat     660
   ttctgtgcaa gacgggagac tacgacggta ggccgttatt actatgctat ggactactgg     720
   ggccaaggga ccacggtcac cgtctcctcc ggaggtggtg gatccgatat caaactgcag     780
   cagtcagggg ctgaactggc aagacctggg gcctcagtga agatgtcctg caagacttct     840
   ggctacacct ttactaggta cacgatgcac tgggtaaaac agaggcctgg acagggtctg     900
   gaatggattg gatacattaa tcctagccgt ggttatacta attacaatca gaagttcaag     960
   gacaaggcca cattgactac agacaaatcc tccagcacag cctacatgca actgagcagc    1020
   ctgacatctg aggactctgc agtctattac tgtgcaagat attatgatga tcattactgc    1080
   cttgactact ggggccaagg caccactctc acagtctcct cagtcgaagg tggaagtgga    1140
   ggttctggtg gaagtggagg ttcaggtgga gtcgacgaca ttcagctgac ccagtctcca    1200
   gcaatcatgt ctgcatctcc aggggagaag gtcaccatga cctgcagagc cagttcaagt    1260
   gtaagttaca tgaactggta ccagcagaag tcaggcacct cccccaaaag atggatttat    1320
   gacacatcca aagtggcttc tggagtccct tatcgcttca gtggcagtgg gtctgggacc    1380
   tcatactctc tcacaatcag cagcatggag gctgaagatg ctgccactta ttactgccaa    1440
   cagtggagta gtaacccgct cacgttcggt gctgggacca agctggagct gaaa           1494
<210> 3
   <211> 124
   <212> PRT
   <213> Artificial Sequence
<220>
   <221> source
   <223> /note="Description of artificial sequence: VH anti CD19"
<400> 3
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
   35 40 45
   Gly Gln Ile Trp Pro Gly Asp Gly Asp Thr Asn Tyr Asn Gly Lys Phe
   50 55 60
   Lys Gly Lys Ala Thr Leu Thr Ala Asp Glu Ser Ser Ser Thr Ala Tyr
   65 70 75 80
   Met Gln Leu Ser Ser Leu Ala Ser Glu Asp Ser Ala Val Tyr Phe Cys
   85 90 95
   Ala Arg Arg Glu Thr Thr Thr Val Gly Arg Tyr Tyr Tyr Ala Met Asp
   100 105 110
   Tyr Trp Gly Gln Gly Thr Thr Val Thr Val Ser Ser
   115 120
<210> 4
   <211> 372
   <212> DNA
   <213> Artificial Sequence
<220>
   <221> source
   <223> /note="Description of artificial sequence: VH anti CD19"
<400> 4
   caggtgcagc tgcagcagtc tggggctgag ctggtgaggc ctgggtcctc agtgaagatt 60
   tcctgcaagg cttctggcta tgcattcagt agctactgga tgaactgggt gaagcagagg 120
   cctggacagg gtcttgagtg gattggacag atttggcctg gagatggtga tactaactac 180
   aatggaaagt tcaagggtaa agccactctg actgcagacg aatcctccag cacagcctac 240
   atgcaactca gcagcctagc atctgaggac tctgcggtct atttctgtgc aagacgggag 300
   actacgacgg taggccgtta ttactatgct atggactact ggggccaagg gaccacggtc 360
   accgtctcct cc 372
<210> 5
   <211> 111
   <212> PRT
   <213> Artificial Sequence
<220>
   <221> source
   <223> /note="Description of artificial sequence: VL anti CD19"
<400> 5
   Asp Ile Gln Leu Thr Gln Ser Pro Ala Ser Leu Ala Val Ser Leu Gly
   1 5 10 15
   Gln Arg Ala Thr Ile Ser Cys Lys Ala Ser Gln Ser Val Asp Tyr Asp
   20 25 30
   Gly Asp Ser Tyr Leu Asn Trp Tyr Gln Gln Ile Pro Gly Gln Pro Pro
   35 40 45
   Lys Leu Leu Ile Tyr Asp Ala Ser Asn Leu Val Ser Gly Ile Pro Pro
   50 55 60
   Arg Phe Ser Gly Ser Gly Ser Gly Thr Asp Phe Thr Leu Asn Ile His
   65 70 75 80
   Pro Val Glu Lys Val Asp Ala Ala Thr Tyr His Cys Gln Gln Ser Thr
   85 90 95
   Glu Asp Pro Trp Thr Phe Gly Gly Gly Thr Lys Leu Glu Ile Lys
   100 105 110
<210> 6
   <211> 333
   <212> DNA
   <213> Artificial Sequence
<220>
   <221> source
   <223> /note="Description of artificial sequence: VL anti CD19"
<400> 6
   gatatccagc tgacccagtc tccagcttct ttggctgtgt ctctagggca gagggccacc 60
   atctcctgca aggccagcca aagtgttgat tatgatggtg atagttattt gaactggtac 120
   caacagattc caggacagcc acccaaactc ctcatctatg atgcatccaa tctagtttct 180
   gggatcccac ccaggtttag tggcagtggg tctgggacag acttcaccct caacatccat 240
   cctgtggaga aggtggatgc tgcaacctat cactgtcagc aaagtactga ggatccgtgg 300
   acgttcggtg gagggaccaa gctcgagatc aaa 333
<210> 7
   <211> 119
   <212> PRT
   <213> Artificial Sequence
<220>
   <221> source
   <223> VH anti CD3
<400> 7
   Asp Ile Lys Leu Gln Gln Ser Gly Ala Glu Leu Ala Arg Pro Gly Ala
   1               5                   10                  15
   Ser Val Lys Met Ser Cys Lys Thr Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Leu Thr Val Ser Ser
           115
<210> 8
   <211> 357
   <212> DNA
   <213> Artificial Sequence
<220>
   <221> source
   <223> /note="Description of artificial sequence: VH anti CD3"
<400> 8
   gatatcaaac tgcagcagtc aggggctgaa ctggcaagac ctggggcctc agtgaagatg 60
   tcctgcaaga cttctggcta cacctttact aggtacacga tgcactgggt aaaacagagg 120
   cctggacagg gtctggaatg gattggatac attaatccta gccgtggtta tactaattac 180
   aatcagaagt tcaaggacaa ggccacattg actacagaca aatcctccag cacagcctac 240
   atgcaactga gcagcctgac atctgaggac tctgcagtct attactgtgc aagatattat 300
   gatgatcatt actgccttga ctactggggc caaggcacca ctctcacagt ctcctca 357
<210> 9
   <211> 106
   <212> PRT
   <213> Artificial Sequence
<220>
   <221> source
   <223> /note="Description of artificial sequence: VL anti CD3"
<400> 9
   Asp Ile Gln Leu Thr Gln Ser Pro Ala Ile Met Ser Ala Ser Pro Gly
   1               5                   10                  15
   Glu Lys Val Thr Met Thr Cys Arg Ala Ser Ser Ser Val Ser Tyr Met
               20                  25                  30
   Asn Trp Tyr Gln Gln Lys Ser Gly Thr Ser Pro Lys Arg Trp Ile Tyr
           35                  40                  45
   Asp Thr Ser Lys Val Ala Ser Gly Val Pro Tyr Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Trp Ser Ser Asn Pro Leu Thr
                   85                  90                  95
   Phe Gly Ala Gly Thr Lys Leu Glu Leu Lys
               100                 105
<210> 10
   <211> 318
   <212> DNA
   <213> Artificial Sequence
<220>
<220>
   <221> source
<400> 10
   gacattcagc tgacccagtc tccagcaatc atgtctgcat ctccagggga gaaggtcacc      60
   atgacctgca gagccagttc aagtgtaagt tacatgaact ggtaccagca gaagtcaggc     120
   acctccccca aaagatggat ttatgacaca tccaaagtgg cttctggagt cccttatcgc     180
   ttcagtggca gtgggtctgg gacctcatac tctctcacaa tcagcagcat ggaggctgaa     240
   gatgctgcca cttattactg ccaacagtgg agtagtaacc cgctcacgtt cggtgctggg     300
   accaagctgg agctgaaa                                                   318
<210> 11
   <211> 15
   <212> PRT
   <213> artificial sequence
<220>
   <221> source
   <223> /note="Description of artificial sequence: anti-CD19 L1"
<400> 11
   Lys Ala Ser Gln Ser Val Asp Tyr Asp Gly Asp Ser Tyr Leu Asn
   1               5                   10                  15
<210> 12
   <211> 7
   <212> PRT
   <213> artificial sequence
<220>
   <221> source
   <223> /note="Description of artificial sequence: anti-CD19 L2"
<400> 12
   Asp Ala Ser Asn Leu Val Ser
   1               5
<210> 13
   <211> 9
   <212> PRT
   <213> artificial sequence
<220>
   <221> source
   <223> /note="Description of artificial sequence: anti-CD19 L3"
<400> 13
   Gln Gln Ser Thr Glu Asp Pro Trp Thr
   1               5
<210> 14
   <211> 5
   <212> PRT
   <213> artificial sequence
<220>
   <221> source
   <223> /note="Description of artificial sequence: anti-CD19 H1"
<400> 14
   Ser Tyr Trp Met Asn
   1               5
<210> 15
   <211> 17
   <212> PRT
   <213> artificial sequence
<220>
   <221> source
   <223> /note="Description of artificial sequence: anti-CD19 H2"
<400> 15
   Gln Ile Trp Pro Gly Asp Gly Asp Thr Asn Tyr Asn Gly Lys Phe Lys
   1 5 10 15
   Gly
<210> 16
   <211> 15
   <212> PRT
   <213> artificial sequence
<220>
   <221> source
   <223> /note="Description of artificial sequence: anti-CD19 H3"
<400> 16
   Arg Glu Thr Thr Thr Val Gly Arg Tyr Tyr Tyr Ala Met Asp Tyr
   1 5 10 15
<210> 17
   <211> 5
   <212> PRT
   <213> artificial sequence
<220>
   <221> source
   <223> /note="Description of artificial sequence: anti-CD3 H1"
<400> 17
   Arg Tyr Thr Met His
   1 5
<210> 18
   <211> 17
   <212> PRT
   <213> artificial sequence
<220>
   <221> source
   <223> /note="Description of artificial sequence: anti-CD3 H2"
<400> 18
   Tyr Ile Asn Pro Ser Arg Gly Tyr Thr Asn Tyr Asn Gln Lys Phe Lys
   1 5 10 15
   Asp
<210> 19
   <211> 10
   <212> PRT
   <213> artificial sequence
<220>
   <221> source
   <223> /note="Description of artificial sequence: anti-CD3 H3"
<400> 19
   Tyr Tyr Asp Asp His Tyr Cys Leu Asp Tyr
   1 5 10
<210> 20
   <211> 10
   <212> PRT
   <213> artificial sequence
<220>
   <221> source
   <223> /note="Description of artificial sequence: anti-CD3 L1"
<400> 20
   Arg Ala Ser Ser Ser Val Ser Tyr Met Asn
   1 5 10
<210> 21
   <211> 7
   <212> PRT
   <213> artificial sequence
<220>
   <221> source
   <223> /note="Description of artificial sequence: anti-CD3 L2"
<400> 21
   Asp Thr Ser Lys Val Ala Ser
   1 5
<210> 22
   <211> 9
   <212> PRT
   <213> artificial sequence
<220>
   <221> source
   <223> /note="Description of artificial sequence: anti-CD3 L3"
<400> 22
   Gln Gln Trp Ser Ser Asn Pro Leu Thr
   1               5

## Description

The present invention relates to a CD19xCD3 bispecific single chain antibody construct for use in a method for the treatment, amelioration or elimination of pediatric acute lymphoblastic leukemia (ALL) in a pediatric ALL patient in the need thereof.

With current childhood ALL treatment, event-free survival rates are about 75%. Therefore, relapse is still frequent. Problems in the management of ALL relapse are the resistance of the leukemic cells and the reduced tolerance of patients to a second round of treatment after having already received intensive frontline therapy, resulting in a lower remission rate as well as a higher incidence of subsequent relapse and an inferior outcome overall. Intensified poly chemotherapy is thus currently essential for induction of a second complete remission. Depending on a variety of prognostic factors, remission may be maintained with chemotherapy and cranial irradiation alone or with intensification of treatment by stem cell transplantation (Henze G, von Stackelberg A, Relapsed acute lymphoblastic leukemia. In: Childhood Leukemias, C-H Pui ed. Cambridge: Cambridge University Press; 2006, p. 473-486).

Although tremendous progress has been made in the treatment of children with acute lymphoblastic leukemia (ALL) (see e.g. Möricke et al., Blood 111 (2008), p. 4477; Moghrabi et al., Blood 109 (2007), p. 896), relapsed ALL is still the fourth most common pediatric malignancy (Gaynon, Cancer 82 (1998), p. 1387). Especially for patients with early bone marrow relapse within three years of diagnosis, therapy for these patients is unsatisfactory and allogeneic stem cell transplantation is the only known curative approach so far. Chemorefractory relapse after hematopoietic stem cell transplantation (HSCT) is associated with a poor prognosis, although post-transplant donor lymphocyte infusions (DLI) might induce remissions in few patients via the induction of a Graft-versus-Leukemia (GvL) effect (Loren et al., BMT 41 (2008), p. 483). Second HSCT can be an option and some long-term survivors have been reported. However, the disease status prior to HSCT is predictive for the post-transplantation outcome and patients with morphological disease or persistant MRD levels of >10⁻⁴ leukemic blasts are known to have a very high risk of relapse and a very poor outcome (Bader et al., J Clin Oncol. 27 (2009), p. 377-84). In light of this, the status of disease prior to second HSCT is of utmost importance and all efforts should be made to achieve another remission. Despite the use of recently introduced chemotherapeutic agents for refractory leukemia (Jeha, Semin Hematol. 46 (2009), 76-88), patients with relapse after HSCT often have chemorefractory disease and these patients are very susceptible to chemotherapy-related toxicity. For these patients, non-chemotherapeutic and less toxic strategies are needed to induce remissions in such patients.

In view of the above-mentioned drawbacks of conventional pediatric ALL therapies, there is still need for improved treatment regimen.

The present invention relates to a CD19xCD3 bispecific single chain antibody construct for use in a method for the treatment, amelioration or elimination of pediatric acute lymphoblastic leukemia (ALL)in a pediatric ALL patient in the need thereof. This immunological approach using T cell engaging antibodies for the first time provides for a non-chemotherapeutic and less toxic treatment for pediatric ALL.

Recently, a phase I study has demonstrated significant clinical activity of the CD19xCD3 bispecific single chain antibody (blinatumomab) in relapsed CD19-positive B-cell non-Hodgkin's lymphoma (NHL) in that impressive partial and complete tumor regressions have been observed (Bargou et al., Science 321 (2008): 974-7). Anderson et al. (Blood J. 80 (1992): 2826-34) describe a bispecific heteroconjugate between an anti-CD19 and anti-CD3 antibody which is said to trigger lysis of acute lymphoblastic leukemia cells.

In a further, still ongoing clinical trial, treatment with the mentioned CD19xCD3 bispecific single chain antibody resulted in the elimination of chemorefratory leukemic cells in non-transplanted MRD-positive adult patients with CD19⁺ ALL.

It has now surprisingly been found in two compassionate uses that said CD19xCD3 bispecific single chain antibody is not only suitable for the treatment of ALL in non-transplanted, adult patients but also of pediatric (or childhood) relapsed ALL refractory to conventional ALL therapy, including chemotherapy and allogeneic hematopoietic stem cell transplantation (HSCT).

Here, the inventors report on the powerful anti-leukemic effect of the CD19xCD3 bispecific antibody in two children (in the following named patient 1 and 2) with B-precursor acute lymphoblastic leukaemia (ALL), who had a chemorefractory relapse after allogeneic hematopoietic stem cell transplantation (HSCT) from matched unrelated and haploidentical donors.

Before treatment with the CD19xCD3 bispecific single chain antibody, patient 1 has been pre-treated with allogenic HSCT and multiple chemotherapies. However, these conventional pediatric ALL therapies failed so that the patient repeatedly relapsed, with the consequence that he had an extremely poor prognosis. In the following, the pediatric ALL patient received 15 microgram/m²/24 hr of the CD19xCD3 bispecific single chain antibody as an continuous infusion for five weeks. During antibody treatment, the patient had an expansion of donor-derived CD8+ T-lymphocytes without any signs of Graft-versus-Host Disease (GvHD). This T-cell expansion was associated with a rapid elimination of the patient's leukemic blasts and 10 days after start of antibody treatment, no blasts could be detected in the patient's bone marrow beyond the minimal residual disease (MRD) detection level of 1 cell out of 10000. The patient remained MRD-negative throughout the antibody treatment. The patient underwent a second stem cell transplantation from his haploidentical mother 2 weeks after end of treatment with the CD19xCD3 bispecific single chain antibody and remained MRD-negative since then (status November 2009).

Fifteen-year old patient 2 was diagnosed with Philadelphia chromosome- and CD19-positive B-precursor ALL in April 2001. After chemotherapy, he received a HSCT from a HLA-identical sibling in October 2001. In 2002, a bone marrow relapsed was diagnosed and another remission was achieved with matinib and chemotherapy. He then received a second HSCT from an HLA-identical unrelated donor in October 2004. In March 2008, a second relapse was diagnosed and he was treated with low-dose chemotherapy and Dasatinib due to resistance to Imatinib. After additional chemotherapy with Clofarabin and Cytosin/Arabinosid, he achieved a molecular remission and received a third allogeneic HSCT from his 3 out of 6 HLA-allele-mismatched haploidentical father with post-transplant treatment with Dasatinib. Due to gastrointestinal bleeding and dilatative cardiomyopathy, Dasatinib was discontinued 5 months after transplantation. In April 2009, a combined central nervous system (CNS) relapse with 7x10⁹/L blasts in the CNS and 3% blasts in the bone marrow was diagnosed. The patient was then treated with Nilotinib, intrathecal chemotherapy and fractionated irradiation of the CNS with 18 Gy. Three months after this treatment, the patient's bone marrow remained MRD-positive at a level of 1.1 x10⁻³ while the CNS was free of blasts. Chimerism analysis of peripheral blood revealed a complete donor-derived hematopoiesis from his haploidentical father.

The patient was then treated with single-agent blinatumomab at 15 µg/m²/day for 4 weeks by continuous infusion without any side effects. A bone marrow aspiration at the end of the treatment showed complete remission with undetectable MRD in the bone marrow below <1x10⁻⁴. As patient 1, patient 2 did not show any signs of GvHD during or after treatment with blinatumomab. He is currently well and attending school.

This data imposingly demonstrate that T-cell engaging bispecific antibodies can induce a powerful Graft-versus-Leukemia (GvL) effect in the absence of GvHD in pediatric patients with relapsed and therapy-refractory B-precursor ALL after allogeneic HSCT. Accordingly, it is preferred in the herein described treatment of pediatric patients that a CD19xCD3 bispecific single chain antibody construct induces a graft-versus-leukemia (GvL) effect. In addition, the treatment is well tolerated by the pediatric patients. In light of this, the methods of this invention provide for a surprisingly improved treatment option for pediatric acute lymphoblastic leukemia (ALL), in particular, for cases in which the pediatric ALL is refractory to conventional pediatric ALL therapy, including chemotherapy and/or allogeneic HSCT. Accordingly, and in a further embodiment of the present invention, the herein employed T-cell engaging bispecific single chain constructs (anti CD19xCD3) can also be employed in pediatric patients who had been under (allogeneic) hematopoietic stem cell transplantation (HSCT). As illustrated herein, even pediatric patients who have a relapse of their ALL after HSCT from a matched unrelated or haploidentical donor and/or pediatric patients who were refractory to chemotherapy can successfully be treated with the methods disclosed herein. The exemplified patients as documented herein showed an impressive anti-leukemic response by the pharmaceutical intervention as disclosed herein and became MRD negative in the absence of any signs of GvHD (graft-versus-host disease) Although the survival of children with ALL improved dramatically over the last decades, relapsed ALL is still the major cause of treatment failure. For patients in 2nd relapse, allogeneic HSCT is the only curative approach so far. One of the main anti-leukemic action of HSCT is the induction of a GvL effect. Unfortunately, the occurrence of GvL is often associated with GvHD, which is still a major cause of morbidity and mortality after HSCT. Therefore, the induction of GvL in the absence of GvHD is the topic of intensive research. One approach for the induction of a GvL effect is post-transplant donor lymphocyte infusions (DLI), which is preferentially used for the treatment of relapsed ALL after HSCT. While DLI is very effective in the treatment of CML (Kolb, Blood 76 (1990), 2462), it is less effective in the post-transplant treatment of relapsed ALL (Loren, BMT 41 (2008), 483) and often leads to the occurrence of GvHD.

The pharmaceutical and medical methods of the present invention provide for a novel approach for the induction of GvL without GvHD. This approach is the in vivo activation of donor-derived lymphocytes after HSCT using low doses of the T-cell engaging CD19xCD3 bispecific single chain antibody, which directs T-lymphocytes against the patients' CD19+ ALL blasts. This antibody has shown impressive anti-lymphoma and anti-leukemic activity in the autologous situation, but was never tested in relapsed pediatric ALL after HSCT. The two pediatric patients described in the following examples had a relapse of their ALL after HSCT from a matched unrelated or haploidentical donor and were refractory to chemotherapy. The patients showed an impressive anti-leukemic response after initiation of treatment and became MRD negative in the absence of any signs of GvHD. The immunological action of the CD19xCD3 bispecific single chain antibody is independent from peptide antigen presentation, and this is most likely the reason that despite of the extensive in vivo donor-derived T-cell expansion, no GvHD was induced. Low doses of the CD19xCD3 bispecific single chain antibody were sufficient to eliminate the ALL blasts in the patients beyond the MRD detection levels. Thus, the T-cell engaging mode of action of this antibody is very different to conventional antibodies, which require much higher doses and which cannot engage T-cells via their Fc part of the antibody molecule due to the lack of Fc receptors on T-cells. Via the CD19xCD3 bispecific single chain antibody, highly effective effector T-cells can become cytotoxic towards ALL blasts without the induction of an alloreactive immune response resulting in GvHD. The inventors decided to perform a second allogeneic HSCT from an haploidentical donor in patient 1 after he became MRD negative. Up to date (November 2009), this patient is still MRD negative.

From the initial clinical experience in these two patients with chemorefractory relapse of ALL post-transplant, it has been concluded that the CD19xCD3 bispecific single chain antibody can induce impressive complete remissions without GvHD. Therefore, treatment with CD19xCD3 bispecific single chain antibody in the pre-transplant setting for immunological reduction of the leukemic load and for the treatment of post-transplant relapses offer new therapeutic chances for pediatric patients with advanced ALL.

The method of the present invention provides for the following major advantages:
1. As demonstrated in the following examples, the administration of the CD19xCD3 bispecific single chain antibody can be used for therapy of pediatric acute lymphoblastic leukemia (ALL) which is relapsed and/or refractory. Not only the CD19xCD3 bispecific single chain antibody can replace conventional pediatric acute lymphoblastic leukemia (ALL) therapies (such as chemotherapy) in pediatric patients non-eligible for allogeneic stem cell transplantation. It can also be used to convert pediatric ALL patients eligible for said transplantation into an MRD negative-status. This aspect of the invention is important in that MRD-negative patients have a lower risk of relapse after transplantation than MRD-positive patients. In the best case scenario, pediatric ALL therapy with CD19xCD3 bispecific single chain antibody makes chemotherapy and/or HSCT superfluous.
2. Pediatric patients with morphological disease or persistent MRD levels of >10⁻⁴ leukemic blasts are known to have a very high risk of relapse and a very poor outcome (Bader et al., J Clin Oncol. 27 (2009), p. 377-84). The children treated with the CD19xCD3 bispecific single chain antibody showed an impressive anti-leukemic response after initiation of treatment and became MRD negative during treatment, in the absence of any signs of GvHD. The pharmaceutical methods of the invention therefore provide a therapeutic approach for the treatment, amelioration or elimination of MRD in pediatric ALL, thereby reducing or even abolishing the risk of a relapse for the patient. The curative potential of allogeneic HSCT depends on the MRD level before transplantation. Treatment with the CD19xCD3 bispecific single chain antibody can be used to convert the mentioned high risk pediatric patients into a MRD-negative status. Accordingly, MRD in refractory childhood ALL can be treated by the CD19xCD3 bispecific single chain antibody for the first time.
3. The pharmaceutical methods of the invention not only show a powerful anti-leukemic effect but they are also less toxic and cause less adverse effects than conventional pediatric ALL therapy, including chemotherapy and/or allogeneic HSCT. So far no long term side effects have been observed after treatment with CD19xCD3 bispecific single chain antibody. In contrast, conventional pediatric ALL therapies are very aggressive and therefore associated with considerable health risks for the pediatric patients. In addition, late effects have been reported after conventional ALL therapy in childhood (see e.g. Hudson MM, Late complications after leukemia therapy. In: Childhood Leukemias, C-H Pui ed. Cambridge: Cambridge University Press; 2006, p. 750-773; Schmoll, Höffken, Possinger: Kompendium Internistische Onkologie, S. 2660 ff.; 4. Auflage, Springer Medizin Verlag Heidelberg; http://www.cancer.gov/cancertopics/pdg/treatment/lateeffects/HealthProfessional). In fact, conventional pediatric ALL treatments use even more aggressive treatment regimens than therapeutic approaches utilized for adult ALL. However, as evident from the about 25% relapse rate in pediatric ALL, not even these aggressive therapies are sufficient to finally cure all patients. In light of this, it is even more surprising that the treatment of refractory and/or relapsed pediatric ALL patients with CD19xCD3 bispecific single chain antibody is able to render said patients into an MRD negative status which offers new therapeutic chances for such high risk patients. This is a result which could not be achieved with conventional pediatric ALL therapy, such as chemotherapy and/or HSCT.
4. For example, Ph+ ALL (bcr/abl) pediatric patients carry a very high risk for a relapse among all patients within the ALL subtypes (see below). Though allogeneic HSCT is currently considered to be the treatment of choice in pediatric Ph+ ALL, approximately one third of the transplanted patients relapse. In addition, as set forth in more detail below, infants (<12 months) with ALL are at higher risk for conventional or standard ALL treatment failure, with the poorest prognosis for those with *MLL* (t(4;11)) gene rearrangements. The results shown for patient 2 in the following examples and the data obtained in the above-mentioned ongoing clinical trial treating adult (non-transplanted) ALL patients, suggest that even Ph+ ALL (bcr/abl) and ALL with *MLL* gene rearrangements can be successfully treated with administration of the CD19xCD3 bispecific single chain antibody. The administration of CD19xCD3 bispecific single chain antibody therefore offers a new therapeutic approach for pediatric Ph+ ALL or ALL with t(4;11) translocations, especially for pediatric ALL patients with minimal residual disease (MRD). This accounts for minimal residual disease (MRD) defined e.g. by PCR or FACS analysis.
5. Due to its very high cytotoxic activitiy, only low doses of CD19xCD3 bispecific single chain antibody are sufficient for the successful treatment of pediatric ALL, which allows the elimination of leukemia cells even in the bone marrow.
6. Shorter duration of treatment with CD19xCD3 bispecific single chain antibody in comparison to conventional pediatric ALL therapy. Conventional pediatric ALL chemotherapy takes usually 2 to 3 years, whereas a very quick response to the CD19xCD3 bispecific single chain antibody could be observed in the pediatric patients shown in the following examples. Moreover, the response is long-lasting: Patient 1 has remained MRD-negative more than 12 months after transplantation; see the following examples.
7. Pediatric ALL patients with relapse often have chemorefractory disease and these patients are very susceptible to chemotherapy-related toxicity. For these patients, treatment with CD19xCD3 bispecific single chain antibody provides for the first time a non-chemotherapeutic and less toxic strategy to induce remissions in such patients.
8. As found in the above-described ongoing clinical trial on ALL in adult patients, treatment with the CD19xCD3 bispecific single chain antibody resulted in the elimination of chemorefratory leukemic cells in non-transplanted MRD-positive adult patients with CD19+ ALL. While in these patients the engaged cytotoxic T-cells were patient-derived, no data existed so far on the use of the CD19xCD3 bispecific single chain antibody after allogeneic HSCT in a setting where the engaged T-cells are donor-derived. Here, the inventors for the first time report on the powerful induction of a GvL effect in the absence of GvHD induced by the CD19xCD3 bispecific single chain antibody engaging donor-derived T-cells in two pediatric patients with chemorefractory relapse of CD19+ALL after allogeneic HSCT.

In summary, treatment with CD19xCD3 bispecific single chain antibody provides for a novel and improved therapy for pediatric ALL, particularly for refractory and/or relapsed pediatric ALL.

In a preferred embodiment of the pharmaceutical methods of the invention, the pediatric or childhood acute lymphoblastic leukemia (ALL) is pediatric B-lineage ALL, preferably pediatric B-precursor acute lymphoblastic leukemia ALL, more preferably pediatric pro-B ALL, pre-B ALL, or common ALL (cALL). Even more preferred the pediatric B-precursor ALL is common ALL (cALL).

The vast majority of pediatric or childhood ALL cases (>85%) are of the B precursor cell phenotype (Schultz et al., Blood 109 (2007): 926-935). Since the CD19xCD3 bispecific single chain antibody described herein is directed against the B cell-associated marker CD19, said antibody is particularly suitable as a therapeutic agent for pediatric B-lineage acute lymphoblastic leukemia, more preferably for pediatric B-precursor ALL. Pediatric B-precursor ALL can be further subdivided into pediatric pro-B ALL, pre-B ALL and common ALL (cALL) (see e.g. Behm F.G., Immunophenotyping. In: Childhood Leukemias, C-H Pui ed. Cambridge: Cambridge University Press; 2006, p. 150-209). Pediatric acute lymphoblastic leukemia (ALL), including pediatric B-precursor acute lymphoblastic leukemia and other types of pediatric B (cell) lineage ALL, and treatments thereof are reviewed e.g. in Pui CH, Clin Adv Hematol Oncol. 4 (2006): 884-6; Pui CH, Evans WE, N Engl J Med 354 (2006): 166-178; Pui CH et al., Lancet 371 (2008): 1030-1043; Pui CH, Jeha S, Nat Rev Drug Discov 6 (2007): 149-165). Further information with respect to pediatric ALL can also be found e.g. under http://www.cancer.gov or http://www.leukemia-lymphoma.org.

From a historical point of view, the Pediatric Oncology Group (POG) and the Children's Cancer Group (CCG) adopted a common set of risk criteria at an international conference supported by the National Cancer Institute (Smith M, et al., J Clin Oncol 14 (1996), 18-24), in 1993. The NCI criteria were based on factors that had an international acceptance and reproducibility: age, initial white blood cell counts (WBC), and the presence of extramedullary disease at diagnosis. To further refine therapy, both POG and CCG have also used additional risk factors that have been shown to have an impact on patient outcomes (e.g. ploidy, blast karyotype, and early morphologic response). In 2000, the CCG and POG joined to form the Children's Oncology Group (COG). This merger allowed analysis of clinical, biologic, and early response data predictive of event-free survival (EFS) in acute lymphoblastic leukemia (ALL) to develop a new classification system and treatment algorithm. From 11 779 children (age, 1 to 21.99 years) with newly diagnosed B-precursor ALL consecutively enrolled by the CCG (December 1988 to August 1995, n = 4986) and POG (January 1986 to November 1999, n = 6793), the study retrospectively analyzed 6238 patients (CCG, 1182; POG, 5056) with informative cytogenetic data (Schultz et al., Blood 109 (2007): 926-935). Four risk groups were defined as very high risk (VHR; 5-year EFS, 45% or below), lower risk (5-year EFS, at least 85%), and standard and high risk (those remaining in the respective National Cancer Institute [NCI] risk groups). VHR criteria included extreme hypodiploidy (fewer than 44 chromosomes), t(9;22) and/or BCR/ABL, and induction failure. Lower-risk patients were NCI standard risk with either t(12;21) (TEL/AML1) or simultaneous trisomies of chromosomes 4, 10, and 17. Even with treatment differences, there was high concordance between the CCG and POG analyses. The COG risk classification scheme is being used for division of B-precursor ALL into lower- (27%), standard- (32%), high- (37%), and very-high- (4%) risk groups based on age, white blood cell (WBC) count, cytogenetics, day-14 marrow response, and end induction minimal residual disease (MRD) by flow cytometry in COG trials.

Currently, risk-based treatment assignment is utilized for childhood or pediatric acute lymphoblastic leukemia (ALL). This approach allows children who historically have a good outcome to be treated with modest therapy and to be spared more intensive and toxic treatment, while allowing children with a historically lower probability of long-term survival to receive more intensive therapy that may increase their chance of cure. It has been found that older children and adolescents (≥10 years) and infants (<12 months) have a less favourable outcome than children aged 1 to 9 years at diagnosis, and more aggressive treatments are generally employed for these patients (Nachman J, Br J Haematol 130 (2005): 166-73). The treatment with CD19xCD3 bispecific single chain antibody provides now an improved, less toxic therapy for such pediatric patient populations, i.e. older children and adolescents (≥10 years) and infants (<12 months), having a less favourable outcome with conventional ALL therapy, such as chemotherapy and/or HSCT.

Successful treatment of children with ALL requires the control of systemic disease (e.g., marrow, liver and spleen, lymph nodes) as well as the prevention or treatment of extramedullary disease, particularly in the central nervous system (CNS). Only 3% of patients have detectable CNS involvement by conventional criteria at diagnosis (≥5 WBC/microliter with lymphoblast cells present). Unless specific therapy is directed toward the CNS, however, 50% to 70% or more of children will eventually develop overt CNS leukemia. Therefore, it is currently recommended that all children with ALL should receive systemic combination chemotherapy together with some form of CNS prophylaxis. At present, most groups treat patients with documented CNS leukemia at diagnosis (>5 WBC/µl with blasts; CNS3), and those with T-cell phenotype and high WBC count at diagnosis, with intrathecal therapy and subsequent cranial radiation. Accordingly, treatment with CD19xCD3 bispecific single chain antibody is preferably carried out in combination with CNS prophylaxis, such as intrathecal therapy and/or cranial radiation.

Conventional or standard treatment for children with ALL is divided into stages: remission induction, consolidation or intensification, and maintenance (or continuation) therapy, with CNS sanctuary therapy generally provided in each stage. An intensification phase of therapy following remission induction is used for all patients. The intensity of both induction therapy and post-induction therapy is determined by the clinical and biologic prognostic factors utilized for risk-based treatment assignment and some type of early response assessment. This assessment may include day 7 and/or day 14 marrow blast percentage, day 8 peripheral blood blast count, and minimal residual disease determinations in bone marrow and/or peripheral blood during or at the end of induction (Pui CH, Evans WE, N Engl J Med 354 (2006): 166-78). The duration of therapy for children with ALL ranges between 2 and 3 years. In contrast, a very quick response to the CD19xCD3 bispecific single chain antibody treatment could be observed in the pediatric patients, as shown in the following examples. In addition, patient 1 is MRD negative up to date (November 2009), indicating that a long-term cure could be achieved.

Subgroups of patients who have a poor prognosis with current standard or conventional therapy may require different treatment. For example, infants with ALL are at higher risk for treatment failure, with the poorest prognosis for those with *MLL* gene rearrangements (Rubnitz JE, et al.: Blood 84 (1994): 570-3; Biondi A, et al., Blood 96 (2000): 24-33; Pui CH, et al., Lancet 359 (2002): 1909-15; Silverman LB, et al.: Cancer 80 (1997): 2285-95). These children are generally treated with regimens designed specifically for infants (Silverman, et al., (1997), loc. cit., Chessells JM, et al., J Haematol 117 (2002): 306-14; Reaman GH, et al., J Clin Oncol 17 (1999): 445-55; Pieters R, et al., Lancet 370 (2007): 240-50). Current regimens for infants employ intensified treatment approaches and may offer improved disease control compared with previous less intensive approaches, but long-term outcome and toxicity are unknown (Reaman (1999), loc. cit.; Pieters (2007), loc. cit.; Kosaka Y, et al., Blood 104 (2004): 3527-34; Hilden JM, et al., Blood 108 (2006): 441-51). Certain children (older than 1 year) with ALL may have a less than 50% likelihood of long-term remission with current therapy (e.g., t[9;22] Philadelphia chromosome-positive ALL, hypodiploid patients, and those with initial induction failure). For these patients, allogeneic bone marrow transplantation from a human leukocyte antigen ( HLA)-matched sibling is considered during first remission (Snyder DS, et al., Leukemia 13 (1999): 2053-8; Aricò M, et al., N Engl J Med 342 (2000): 998-1006; Schrauder A, et al., J Clin Oncol 24 (2006): 5742-9). HLA-matched sibling donor transplant, however, has not been proven to be of benefit in patients defined as high-risk solely by WBC count, gender, and age (Ribera JM, et al., J Clin Oncol 25 (2007): 16-24).

Since myelosuppression and generalized immunosuppression are an anticipated consequence of both leukemia and its treatment with chemotherapy, patients must be closely monitored during conventional pediatric ALL treatment. Adequate facilities must be immediately available both for hematologic support and for the treatment of infectious and other complications throughout all phases of leukemia treatment. Approximately 1% of patients die during induction therapy and another 1% to 3% die during first remission from treatment-related complications (Christensen MS, et al., Br J Haematol 131 (2005): 50-8).

Treatment with CD19xCD3 bispecific single chain antibody provides for an alternative and less toxic therapy for pediatric ALL, particularly for refractory and/or relapsed pediatric ALL. Said treatment avoids the drawbacks of conventional childhood ALL therapies, such as treatment failures, toxicity and long-term adverse effects. It is therefore a highly efficient but less toxic and health-risk alternative to chemotherapy and/or allogeneic HSCT.

Accordingly, in another embodiment of the pharmaceutical methods of the invention, said acute lymphoblastic leukemia (ALL) is refractory and/or relapsed ALL.

The pediatric ALL may be refractory to chemotherapy or allogeneic hematopoietic stem cell transplantation (HSCT) or to chemotherapy and allogeneic hematopoietic stem cell transplantation (HSCT). The ALL may be relapsed ALL or relapsed ALL refractory to conventional ALL therapy, including chemotherapy and/or allogeneic hematopoietic stem cell transplantation (HSCT). However, it is also within the ambit of the invention that the ALL is a newly diagnosed ALL. In this setting, the treatment by the CD19xCD3 bispecific single chain antibody may be used as a first (frontline) therapy, either alone or in combination with HSCT.

The term "refractory pediatric ALL" as used herein means resistance of the pediatric ALL to conventional or standard pediatric ALL therapy, such as chemotherapy and/or HSCT. Currently, the relapse rate in pediatric ALL is about 25%. Put in other words: The conventional or standard pediatric ALL therapy is not able to ultimately cure all pediatric patients.

The term "relapsed pediatric ALL" as used herein denotes the return of signs and symptoms of the ALL disease after a pediatric patient has enjoyed a remission. For example, after conventional ALL treatment using chemotherapy and/or HSCT, a pediatric ALL patient may go into remission with no sign or symptom of the ALL, remains in remission for a couple of years, but then suffers a relapse and has to be treated once again for ALL.

Pediatric ALL patients with relapse often have chemorefractory disease. These patients are very susceptible to chemotherapy-related toxicity which can be avoided by treatment with the CD19xCD3 bispecific single chain antibody.

The term "standard therapy" or "conventional therapy" as used herein refers to pediatric ALL therapy using chemotherapy and/or HSCT. Pediatric acute lymphoblastic leukemia (ALL), including pediatric B-precursor acute lymphoblastic leukemia and other types of pediatric B (cell) lineage ALL, and treatments thereof are reviewed e.g. in Pui CH, Clin Adv Hematol Oncol. 4 (2006): 884-6; Pui CH, Evans WE, N Engl J Med 354 (2006): 166-178; Pui CH et al., Lancet 371 (2008): 1030-1043; Pui CH, Jeha S, Nat Rev Drug Discov 6 (2007): 149-165); Henze G, von Stackelberg A, Relapsed acute lymphoblastic leukemia. In: Childhood Leukemias, C-H Pui ed. Cambridge: Cambridge University Press; 2006, p. 473-486). Further information with respect to pediatric ALL can also be found e.g. under http://www.cancer.gov or http://www.leukemia-lymphoma.org.

The term "bispecific single chain antibody" or "single chain bispecific antibody" or related terms in accordance with the present invention mean antibody constructs resulting from joining at least two antibody variable regions in a single polypeptide chain devoid of the constant and/or Fc portion(s) present in full immunoglobulins. The bispecific single chain antibody as referred to herein is functional, i.e. cytotoxically active, as a monomer and therefore clearly distinguishable from diabodies or tandabs described in the art which are functional only as dimers or multimers. A "linker" as used herein connects V domains of the same specificity, whereas a "spacer" as used herein connects V domains of different specificities. For example, a bispecific single chain antibody may be a construct with a total of two antibody variable regions, for example two VH regions, each capable of specifically binding to a separate antigen, and connected with one another through a short (usually less than 10 amino acids) synthetic polypeptide spacer such that the two antibody variable regions with their interposed spacer exist as a single contiguous polypeptide chain. Another example of a bispecific single chain antibody may be a single polypeptide chain with three antibody variable regions. Here, two antibody variable regions, for example one VH and one VL, may make up an scFv, wherein the two antibody variable regions are connected to one another via a synthetic polypeptide linker, the latter often being genetically engineered so as to be minimally immunogenic while remaining maximally resistant to proteolysis. This scFv is capable of specifically binding to a particular antigen, and is connected to a further antibody variable region, for example a VH region, capable of binding to a different antigen than that bound by the scFv. Yet another example of a bispecific single chain antibody may be a single polypeptide chain with four antibody variable regions. Here, the first two antibody variable regions, for example a VH region and a VL region, may form one scFv capable of binding to one antigen, whereas the second VH region and VL region may form a second scFv capable of binding to another antigen. Within a single contiguous polypeptide chain, individual antibody variable regions of one specificity may advantageously be separated by a synthetic polypeptide linker as described above, whereas the respective scFvs may advantageously be separated by a short polypeptide spacer as described above. Non-limiting examples of bispecific single chain antibodies as well as methods for producing them are shown in WO 99/54440, WO 2004/106381, WO 2007/068354, Mack, J. Immunol. (1997), 158, 3965-70; Mack, PNAS, (1995), 92, 7021-5; Kufer, Cancer Immunol. Immunother., (1997), 45, 193-7; Löffler, Blood, (2000), 95, 6, 2098-103; Brühl, J. Immunol., (2001), 166, 2420-2426.

As used herein, "CD3" denotes an antigen that is expressed on T cells, preferably human T cells as part of the multimolecular T cell receptor complex, the CD3 consisting of five different chains: CD3-epsilon, CD3-gamma, CD3-delta, CD3-eta and CD3 zeta. Clustering of CD3 on T cells e.g. by anti-CD3 antibodies leads to T cell activation similar to the binding of an antigen but independent from the clonal specificity of the T cell subset. Thus, the term "CD19xCD3 bispecific single chain antibody" as used herein relates to a CD3-specific construct capable of binding to the human CD3 complex expressed on human T cells and capable of inducing elimination/lysis of target cells, wherein such target cells carry/display an antigen which is bound by the other, non-CD3-binding portion of the bispecific single chain antibody. Binding of the CD3 complex by CD3-specific binders (e.g. a bispecific single chain antibody as administered according to the pharmaceutical methods of the invention) leads to activation of T cells as known in the art; see e.g. WO 99/54440 or WO 2007/068354. Accordingly, a construct appropriate for the pharmaceutical methods of the invention is advantageously able to eliminate/lyse target cells in vivo and/or in vitro. Corresponding target cells comprise cells expressing a tumor antigen, such as CD19, which is recognized by the second specificity (i.e. the non-CD3-binding portion of the bispecific single chain antibody) of the mentioned construct. Preferably, said second specificity is for human CD19 which has already been described in WO 99/54440, WO 2004/106381 or WO 2007/068354. According to this embodiment, each antigen-specific portion of the bispecific single chain antibody comprises an antibody VH region and an antibody VL region. An advantageous variant of this bispecific single chain antibody is from N terminus to C terminus:
V_{L}(CD19)-V_{H}(CD19)-V_{H}(CD3)-V_{L}(CD3).

Within the meaning of the invention, the term "specifically binding" or related terms such as "specificity" is/are to be understood as being characterized primarily by two parameters: a qualitative parameter (the binding epitope, or *where* an antibody binds) and a quantitative parameter (the binding affinity, or *how strongly* this antibody binds where it does). Which epitope is bound by an antibody can advantageously be determined by e.g. FACS methodology, ELISA, peptide-spot epitope mapping, or mass spectroscopy. The strength of antibody binding to a particular epitope may advantageously be determined by e.g. known Biacore and/or ELISA methodologies. A combination of such techniques allows the calculation of a signal:noise ratio as a representative measure of binding specificity. In such a signal:noise ratio, the signal represents the strength of antibody binding to the epitope of interest, whereas the noise represents the strength of antibody binding to other, non-related epitopes differing from the epitope of interest. A signal:noise ratio of, for example at least 50, but preferably about 80 for a respective epitope of interest as determined e.g. by Biacore, ELISA or FACS may be taken as an indication that the antibody evaluated binds the epitope of interest in a specific manner, i.e. is a "specific binder". The term "binding to/interacting with" may also relate to a conformational epitope, a structural epitope or a discountinuous epitope consisting of two or even more regions of the human target molecules or parts thereof. A conformational epitope is defined by two or more discrete amino acid sequences separated in the primary sequence which come together on the surface of the molecule when the polypeptide folds to the native protein (Sela, (1969) Science 166, 1365 and Laver, (1990) Cell 61, 553-6). The term "discontinuous epitope" means non-linear epitopes that are assembled from residues from distant portions of the polypeptide chain. These residues come together on the surface of the molecule when the polypeptide chain folds into a three-dimensional structure to constitute a conformational/structural epitope.

The term "treatment" as used herein means in the broadest sense medical procedures or applications that are intended to relieve illness. In the present case, the administration of the CD19xCD3 bispecific single chain antibody (prepared for administration to a pediatric ALL patient) as described herein is for the treatment, amelioration or elimination of the pediatric ALL disease.

The term "amelioration" as used herein is synonymous with improvement. If a pediatric ALL patient's condition shows amelioration, the patient is clearly better - there is some improvement in her or his clinical condition. For example, it may be an improvement in the ALL patient's condition, if a stabilization of the ALL disease can be achieved, i.e. the ALL disease is no longer progressive. This disease stage is also termed stable disease. Amelioration can also be an improvement of the MRD status of the pediatric ALL patient. For example, before treatment with the CD19xCD3 bispecific single chain antibody, 100 leukemia cells per 10⁴ bone marrow cells may be detectable in the pediatric ALL patient. Due to the CD19xCD3 bispecific single chain antibody treatment, the number of leukemia cells may be reduced to 10 leukemia cells or even less leukemia cells (e.g. less than one leukemia cell) per 10⁴ bone marrow cells in this exemplary case.

The term "elimination" as used herein means the removal of leukemic cells from the body of a pediatric ALL patient. As shown in the following example, administration of the CD19xCD3 bispecific single chain antibody is able to convert MRD positive acute lymphoblastic leukemia (ALL) into an MRD negative status, i.e. a status in which no MRD is detectable (<10⁻⁴, i.e. less than 1 leukemia cell per 10⁴ bone marrow cells detectable). In this case, a complete molecular remission is reached.

The term "administration" as used herein means administration of a therapeutically effective dose of the aforementioned CD19xCD3 bispecific single chain antibody (preferably the one shown in SEQ ID NO.1) to an individual, i.e. a human pediatric patient. By "therapeutically effective amount" is meant a dose that produces the effects for which it is administered, i.e. sufficient to kill acute lymphoblastic leukemia cells. Preferably, the administered dose of the CD19xCD3 bispecific single chain antibody leads to the elimination of all acute lymphoblastic leukemia cells from the body of the pediatric patient, resulting in an MRD-negative ALL status, as defined herein. The exact dose will depend on the purpose of the treatment, and will be ascertainable by one skilled in the art using known techniques. The attending physician and clinical factors will determine the dosage regimen. As is well known in the medical arts, dosages for any one pediatric patient depends upon many factors, including the pediatric patient's size, body surface area, age, body weight, the particular compound to be administered, sex, time and route of administration, general health status, and other drugs being administered concurrently.

A typical dose can be, for example, in the ranges set forth in the embodiments of the methods of the invention and the appended examples; however, doses below or above this exemplary range are envisioned, especially considering the aforementioned factors.

The term "continuous infusion" refers to an infusion which is allowed to proceed permanently over a time period, i.e. without interruption. "Continuous infusion" refers to a permanently administered infusion. Accordingly, in the context of the invention, the terms "permanent" and "continuous" are used as synonyms. Within the meaning of the invention, e.g. the term "administered by continuous infusion for (at least) 4 weeks" or the like denote(s) a situation in which the CD19xCD3 bispecific single chain antibody used in the pharmaceutical methods according to the invention is continuously administered to the body of a pediatric patient over a period of (at least) 4 weeks in a sustained, constant fashion throughout the entire duration required in the pharmaceutical means and methods of the invention. Continuous administration schemes of the CD19xCD3 bispecific single chain antibody are described in more detail in WO 2007/068354. An interruption of the introduction of CD19xCD3 bispecific single chain antibody is avoided, that is to say a transition from a state in which this antibody is being administered to the body of the pediatric patient to a state in which this antibody is no longer being administered to the body of the pediatric patient does not, or does not significantly occur over the entire duration of administration required by the pharmaceutical methods of the invention for other reasons than replenishing the supply of CD19xCD3 bispecific single chain antibody being administered or medical interventions which become necessary and the like. In as far as such necessary replenishing leads to a temporary interruption of the introduction of the antibody administered, such administration is still to be understood as being "uninterrupted" or "permanent" in the sense of the pharmaceutical methods according to the invention. In most cases, such replenishing will generally be of such a short duration that the time during which antibody is not being introduced into the body of the pediatric patient will be vanishingly small when compared to the time planned for the overall administration regimen according to the pharmaceutical methods according to the invention. In accordance with the invention, one treatment cycle can be understood as continuous infusion of the CD19xCD3 bispecific single chain antibody to the ALL patient for a period of (at least) 4 weeks, followed by a 2-week treatment-free interval. The term "at least" as used herein means that the continuous infusion can also be carried out for a time period longer than 4 weeks, e.g. for 5, 6, 7 or 8 weeks or even longer, followed by a 2-week treatment-free interval. It may be the case that upon MRD staging of the treated pediatric patient(s) after an (at least) 4 week-continuous administration (or one treatment cycle), a minimal or partial response but no MRD negativity could be achieved. Under such circumstances, the continuous administration may be extended by additional one, two, three, four, five or even up to ten treatment cycles in order to achieve a better therapeutic result, e.g. a complete hematologic, or even complete molecular response. Preferably, said complete molecular response is MRD negativity (as defined hereinafter) which decreases the risk of recurrence of the ALL disease. For example, it has been found that MRD-negativity or low MRD levels in pediatric ALL patients before allogenic HSCT reduces the risk of a relapse (Bader P, et al., J Clin Oncol 27 (2009): 377-384). As shown in the following examples, MRD negativity can be achieved by treatment of the pediatric ALL patients with the CD19xCD3 bispecific single chain antibody.

Therefore, in one embodiment of the pharmaceutical methods of the invention, the one or more treatment cycle(s), in which the CD19xCD3 bispecific single chain antibody is continuously administered to the pediatric ALL patient, is/are followed by allogeneic HSCT. Put in other words: In this preferred embodiment, the method of the invention is prior to allogeneic stem cell transplantation (HSCT) in order to convert the MRD positive ALL into an MRD negative status. In this way, the risk of a relapse is significantly decreased.

In another embodiment of the method of the invention, the method is after allogeneic hematopoietic stem cell transplantation (HSCT).

The transplantation procedure may be followed by one or further treatment cycle(s) with CD19xCD3 bispecific single chain antibody. This embodiment is important in cases of relapsed pediatric ALL, where the patients have received chemotherapy and HSCT. Due to the failure of these conventional therapies, the disease may relapse and now be cured by the administration of said antibody. In addition, treatment by the CD19xCD3 bispecific single chain antibody can be used as consolidation therapy after HSCT in order to avoid another relapse of the ALL disease.

The following examples provide also data on the use of CD19xCD3 bispecific single chain antibody after allogeneic HSCT where the engaged T cells are donor-derived. A powerful induction of a Graft-versus-leukemia (GvL) effect in the absence of Graft-versus-Host Disease (GvHD) induced by the treatment with CD19xCD3 bispecific single chain antibody in two patients with chemorefractory relapse of CD19+ALL after allogeneic hematopoietic HSCT could be observed. Therefore, in another preferred embodiment of the pharmaceutical methods of the invention, the pediatric ALL patient can be treated with CD19xCD3 bispecific single chain antibody after she/he has received an allogeneic HSCT.

In a best case scenario it is envisaged that the treatment of pediatric ALL patients with the CD19xCD3 bispecific single chain antibody is able to replace conventional pediatric ALL therapy, such as chemotherapy and/or allogeneic hematopoietic stem cell transplantation (HSCT).

As shown in the following examples, the treatment of the pediatric acute lymphoblastic leukemia (ALL) patient with the CD19xCD3 bispecific single chain antibody is able to eliminate lymphoblastic acute leukemia cells from the body of the patients below detection limit. Preferably, the major therapeutic goal of the administration of the CD19xCD3 bispecific single chain antibody, either alone or in combination with allogeneic HSCT, to an ALL pediatric patient is the conversion of an MRD-positive status into an MRD-negative status resulting in leukemia-free survival, as defined herein below. As demonstrated herein, the MRD-positive pediatric ALL patients turned MRD negative after the first treatment cycle in which the CD19xCD3 bispecific single chain antibody has been continuously administered for five weeks (patient 1) or four weeks (patient 2). In patient 1, the treatment cycle has been followed by an haploid HSCT. As of November 2009, this patient remains in MRD-negative complete remission, i.e. tumor free.

Continuing uninterrupted administration of the CD19xCD3 bispecific single chain antibody in the manner of the pharmaceutical methods according to the invention for longer periods of time allows the advantageous T cell activation mentioned in the examples to exert its effect for long enough to advantageously clear all acute lymphoblastic leukemia cells from the body. Since the rate of uninterruptedly administered bispecific single chain antibody is kept low, application of therapeutic agent may be continued longer without risk of deleterious side effects for the patient.

The CD19xCD3 bispecific single chain antibody as used herein is advantageously to be prepared in the form of a pharmaceutical composition for administration to a human pediatric patient diagnosed with acute lymphoblastic leukemia (ALL). Said ALL may be a newly diagnosed ALL, an ALL refractory to chemotherapy and/or allogeneic hematopoietic stem cell transplantation (HSCT), a relapsed ALL or a relapsed ALL refractory to chemotherapy and/or allogeneic hematopoietic stem cell transplantation (HSCT).

In a preferred embodiment of the pharmaceutical methods of the invention, said pediatric acute lymphoblastic leukemia (ALL) is pediatric B-lineage acute lymphoblastic leukemia (ALL), preferably pediatric B-precursor acute lymphoblastic leukemia. The vast majority of pediatric ALL cases (>85%) are of the B precursor cell phenotype. Several classifications of B-lineage ALL have been proposed (see e.g. Schultz et al., Blood 109 (2007): 926-935). In order to modulate the intensity of therapy relative to a patient's risk of relapse, B precursor ALL patients are currently stratified into "low", "standard", "high" or "very high" risk groups using laboratory and clinical parameters: patient age, sex, white blood cell count (WBC) at disease presentation, and the presence or absence of specific cytogenetic abnormalities. The frequently recurring genetic abnormalities that help define these risk groups include for example: t(12;21)[TEL-AML1]; t(1;19;)[E2A-PBX]; t(4;11)[AF4-MLL]; t(9;22)[BCR-ABL]; hyperdiploidy (or trisomy of chromosomes 4, 10, and 17), and hypodiploidy; see e.g. Schultz et al.; Bader et al., loc. cit. Using the data from various studies, a classification system has been developed and implemented as COG AALL03B1 (Classification of Acute Lymphoblastic Leukemia) (Raetz et al., Personalized Med. 2 (2005), 349-361; Schultz et al., Blood 109 (2007): 926-935). Since the CD19xCD3 bispecific single chain antibody described herein is directed against the B cell-associated marker CD19, said antibody is particularly suitable as a therapeutic agent for pediatric B-lineage acute lymphoblastic leukemia, preferably for pediatric B-precursor ALL's. Pediatric B-precursor ALL's can be further subdivided into pediatric pro-B ALL, pre-B ALL and common ALL (cALL). As shown in the following examples, the treatment according to the methods of the invention of the seven year aged patient 1 with common ALL being refractory against any treatment and therefore prone to be fatal, resulted not only in complete hematologic remission but in complete molecular remission. Put in other words: no minimal residual disease could be detected any more in this patient after treatment with CD19xCD3 bispecific single chain antibody. Particularly preferred, the pediatric acute lymphoblastic leukemia (ALL) is B-precursor ALL, more preferably cALL. Importantly, the CD19xCD3 bispecific single chain antibody is able to kill not only ALL cells having TCR or immunoglobulin rearrangements, but also ALL cells with various other cytogenetic abnormalities: For example, it has been found in patient 2 described in the following examples as well as in adult ALL patients that said antibody is able to treat ALL characterized by immunoglobulin or TCR rearrangements, t(4;11) translocations or bcr/abl fusion transcripts (Ph+). Particularly Ph+ ALL and ALL with t(4;11) translocations have been reported to be extremely difficult to treat by conventional ALL therapy but could be successfully treated by the CD19xCD3 bispecific single chain antibody. The mentioned data indicate that the CD19xCD3 bispecific single chain antibody is able to treat various forms of ALL including e.g. ALL characterized by t(12;21)[TEL-AML1]; t(1;19;)[E2A-PBX]; t(4;11)[AF4-MLL]; t(9;22)[BCR-ABL]; hyperdiploidy (or trisomy of chromosomes 4, 10, and 17), hypodiploidy as well as immunoglobulin or TCR rearrangements; see also Table 1.

The diagnosis of pediatric ALL is based on morphologic, cytochemical, and immunologic features of the cells, including lymphoblast morphology on Wright-Giemsastained bone marrow smears, positive staining for terminal deoxynucleotide transferase (TdT), negative staining for myeloperoxidase, and cell surface expression of 2 or more B-cell-precursor lymphoid differentiation antigens. Immunophenotyping is described, for instance, by Behm F.G. (Immunophenotyping. In: Childhood Leukemias, C-H Pui ed. Cambridge: Cambridge University Press; 2006, p. 150-209), and can be performed e.g. by indirect immunofluorescence, immunohistochemistry and/or flow cytometry.

The term "patient" as used herein refers to a human patient. The term "pediatric ALL" or "pediatric ALL patient" as referred to herein denotes children aged from one month to 18 years. The indicated age is to be understood as the age of the children at diagnosis of the ALL disease. The children may be more specifically sub-grouped into infants (1 to 12 months of age), younger children aged 1 to 9 years, and older children and adolescents (≥10 to 18 years of age). As used herein, a time interval which is defined as "X to Y" equates with a time interval which is defined as "between X and Y". Both time intervals specifically include the upper limit and also the lower limit. This means that for example a time interval "from one month to 18 years" includes "one month" and "18 years". Preferably, the patient to be treated in accordance with the present invention is at most 18 years old (including patients aged 18 years).

As shown in the following examples, patient 1 was aged seven years when treated with the CD19xCD3 bispecific single chain antibody, with ALL diagnosed at two years of age. Patient 1 was aged fifteen years when treated with the CD19xCD3 bispecific single chain antibody, with ALL diagnosed in 2001.

The above definitions apply mutatis mutandis to the term "pediatric acute lymphoblastic leukemia (ALL)", "childhood ALL", or the like. For example, pediatric or childhood ALL is to be understood as ALL diagnosed in a pediatric patient aged between 1 month (including 1 month) and 18 years (including 18 years).

While the CD19xCD3 bispecific single chain antibody as used herein may be administered per se, administration in a pharmaceutically acceptable carrier is preferred. Examples of suitable pharmaceutical carriers are well known in the art and include phosphate buffered saline solutions, water, liposomes, various types of wetting agents, sterile solutions, etc. Compositions comprising such carriers can be formulated by well known conventional methods. These pharmaceutical compositions can be administered to the pediatric patient at a suitable dose. The dosage regimen will be determined by the attending physician and clinical factors. As is well known in the medical arts, dosages for any one pediatric patient depends upon many factors, including the patient's size, body surface area, age, body weight, the particular compound to be administered, sex, time and route of administration, general health, and other drugs being administered concurrently. Preparations for parenteral administration include sterile aqueous or non-aqueous solutions, or suspensions. Examples of non-aqueous solvents are propylene glycol, polyethylene glycol, and injectable organic esters such as ethyl oleate. Aqueous carriers include water, aqueous solutions, or suspensions, including saline and buffered media. Parenteral vehicles include sodium chloride solution, Ringer's dextrose, dextrose and sodium chloride, or lactated Ringer's. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the composition might comprise proteinaceous carriers, like, e.g., serum albumine or immunoglobuline, preferably of human origin. It is envisaged that the composition might comprise, in addition to the proteinaceous bispecific single chain antibody further biologically active agents, depending on the intended use of the pharmaceutical composition. Such agents might be agents acting as cytostatica, agents preventing hyperurikemia, agents inhibiting immune reactions (e.g. corticosteroids, FK506), drugs acting on the circulatory system and/or agents such as T-cell co-stimulatory molecules or cytokines known in the art. For example, treatment with CD19xCD3 bispecific single chain antibody can be carried out in combination with intrathecal chemotherapy as CNS prophylaxis, corticoids, and/or allopurinol.

Preferably, the CD19xCD3 bispecific single chain antibody as defined herein is formulated in a buffer, a stabilizer and a surfactant. The buffer may be a phosphate, citrate, succinate or acetate buffer. The stabilizer may be (an) amino acid(s) and/or a sugar. The surfactants may be detergents, PEGs, or the like. More preferably, the CD19xCD3 bispecific single chain antibody as defined herein is formulated in citrate, lysine, trehalose and Tween 80. As a diluent for the pharmaceutical composition of the invention, isotonic saline and Tween 80 is preferred.

Preferably, in the pharmaceutical methods of the invention, the pharmaceutical composition is to be prepared for administration to a human pediatric patient diagnosed with acute lymphoblastic leukemia (ALL).

The success of the CD19xCD3 bispecific single chain antibody therapy may be monitored by established standard methods for the respective disease entities: For B cell ALL therapy, Fluorescence Activated Cell Sorting (FACS), bone marrow aspiration and various leukemia specific clinical chemistry parameters and other established standard methods known in the art may be used. Methods and means for the determination of the minimal residual disease (MRD) status are described herein.

Cytotoxic activity of the CD19xCD3 bispecific single chain antibody can be detected by methods known in the art and methods as illustrated e.g. in WO 99/54440, WO 2004/106381, WO 2007/068354.

In a preferred embodiment of the pharmaceutical methods of the invention, the B-precursor ALL of the pediatric patient(s) is relapsed and/or refractory to treatment. With current childhood ALL treatment, event-free survival rates are about 75%. Therefore, 25% of the patients relapse inspite of the toxic and health-risky treatment. Problems in the management of ALL relapse are the resistance of the leukemic cells and the reduced tolerance of pediatric patients to a second round of treatment after having already received intensive frontline therapy, resulting in a lower remission rate as well as a higher incidence of subsequent relapse and an inferior outcome overall. Intensified poly chemotherapy is then essential for induction of a second complete remission (Henze G, von Stackelberg A, Relapsed acute lymphoblastic leukemia. In: Childhood Leukemias, C-H Pui ed. Cambridge: Cambridge University Press; 2006, p. 473-486). The term "refractory to chemotherapy and/or allogenic stem cell transplantation" as used herein means that the pediatric ALL patients is resistant to these therapies and therefore relapsed after conventional ALL treatment. It is also envisaged that pediatric patients who relapsed after treatment with the CD19xCD3 bispecific single chain antibody receive one or more further treatment cycles with said antibody in order to render the treated children MRD negative. Said patients may then for instance receive a second allogeneic HSCT.

As shown in the following examples, the pharmaceutical methods of the invention are particularly suitable for the treatment of pediatric patients resistant to conventional ALL therapies. Though the shown pediatric patients have been heavily pre-treated with both chemotherapy and allogenic stem cell transplantation, the patients relapsed several times. The patients therefore had an extremely poor prognosis. However, after treatment with the CD19xCD3 bispecific single chain antibody, the patients were MRD negative, i.e. showed a complete molecular remission. Put in other words: The treatment eliminated acute lymphoblastic leukemia cells from the pediatric patients' body below detection limit. Most importantly, the bone marrow of the pediatric patients have been cleared from leukemic cells.

In a preferred embodiment of the pharmaceutical methods of the invention, the acute lymphoblastic leukemia (ALL) of the pediatric patient(s) is refractory to chemotherapy and/or allogeneic HSCT with respect to MRD. Put in other words: the MRD in the pediatric ALL patients is resistant to chemotherapy and/or allogeneic HSCT.

It is envisaged that the pharmaceutical methods of the invention are suitable also for the treatment of newly diagnosed pediatric ALL in that it provides for an alternative to conventional pediatric ALL treatment regimens, such as chemotherapy and/or allogeneic HSCT.

In a further preferred embodiment, said acute lymphoblastic leukemia (ALL) relapses within three years of diagnosis, preferably within two years of diagnosis, even more preferably within one year of diagnosis. Preferably, said relapse is a bone marrow relapse.

The term "chemotherapy" as used herein denotes chemotherapy used for the treatment of pediatric acute lymphoblastic leukemia (ALL). Chemotherapy is the initial treatment of choice for pediatric ALL (see e.g. Pui CH, Jeha S, Nat Rev Drug Discov 6 (2007): 149-165; Schmiegelow K, Gustaffson G. Acute lymphoblastic leukemia. In: Cancer in Children: Clinical Management. Voute PA, Barret A, Stevens MCG, Caron HN (eds). London, UK, Oxford University Press, 2005, p. 138-170; Schmoll, Höffken, Possinger, loc. cit.). Most pediatric ALL patients end up receiving a combination of different treatments. In the treatment of pediatric ALL, there are no surgical options, due to the body-wide distribution of the malignant cells. In general, cytotoxic chemotherapy for pediatric ALL combines multiple anti-leukemic drugs in various combinations. Chemotherapy for pediatric ALL consists of three phases: remission induction, intensification, and maintenance therapy. Chemotherapy is also indicated to protect the central nervous system from leukemia. The aim of remission induction is to rapidly kill most tumor cells and get the pediatric patient into complete haematological remission. This is defined as the presence of less than 5% leukemic blasts in the bone marrow (as determined by light microscopy), For example, Clofarabine, Cyclophosphamide, VP16, Amsacrine, Prednisone, Melphalan, or Cytarabine, alone or in combination, is used to induce remission. Intensification uses high doses of intravenous multidrug chemotherapy to further reduce tumor burden. Typical intensification protocols use vincristine, cyclophosphamide, cytarabine, daunorubicin, etoposide, thioguanine or mercaptopurine given as blocks in different combinations. Since ALL cells sometimes penetrate the Central Nervous System (CNS), most protocols include delivery of chemotherapy into the CNS fluid commonly known as intrathecal chemotherapy. Some tumor centers deliver the drug through Ommaya reservoir (a device surgically placed under the scalp and used to deliver drugs to the CNS fluid and to extract CNS fluid for various tests). Other centers perform multiple lumbar punctures as needed for testing and treatment delivery. Intrathecal methotrexate or cytarabine is usually used for this purpose. The aim of maintenance therapy is to kill any residual cell that was not killed by remission induction, and intensification regimens. Although such cells are few, they will cause relapse if not eradicated. For this purpose, daily oral mercaptopurine, once weekly oral methotrexate, once monthly 5-day course of intravenous vincristine an. The length of maintenance therapy is 3 years for boys, 2 years for girls and adults. Nervous system relapse is treated with intrathecal administration of hydrocortisone, methotrexate, and cytarabine. As the chemotherapy regimens can be intensive and protracted (often about 2 years in case of the GMALL UKALL, HyperCVAD or CALGB protocols; about 3 years for males on COG protocols), many patients have an intravenous catheter inserted into a large vein (termed a central venous catheter or a Hickman line), or a Portacath (a cone-shaped port with a silicone nose that is surgically planted under the skin, usually near the collar bone). However, chemotherapy remains a highly toxic procedure, in particular for pediatric patients.

Patients can experience a recurrence of ALL after initial therapy and/or become refractory to chemotherapy following treatment. Pediatric ALL patients who are refractory to chemotherapy have a markedly poor prognosis. In particular, the prognosis of pediatric patients with Ph+ ALL or ALL with t(4;11) translocations treated only with chemotherapy is poor. Since the method of the invention is capable of rendering the pediatric ALL patients MRD-negative, it is particularly useful for the treatment of ALL patients refractory to chemotherapy and/or allogeneic HSCT. In light of this, the term "refractory to chemotherapy and/or allogeneic HSCT" as used herein denotes resistance of the acute lymphoblastic leukemia cells to chemotherapy and/or allogeneic HSCT.

The term "allogeneic hematopoietic stem cell transplantation (HSCT)" as used herein means allogeneic hematopoietic stem cell transplantation (HSCT) or bone marrow transplantation which is a medical procedure in the field of hematology and oncology that involves transplantation of hematopoietic stem cells (HSC). It is most often performed for patients with diseases of the blood or bone marrow, or certain types of cancer, such as ALL. Most recipients of HSCTs are leukemia (e.g. ALL) patients who would benefit from treatment with high doses of chemotherapy or total body irradiation. Allogeneic HSCT in children with ALL is described e.g. in Schrauder A, et al. (Bone Marrow Transplantation 41 (2008): Suppl2 S71-74). However, allogeneic stem cell transplantation remains a risky procedure, in particular for pediatric patients.

The term "eligible for allogeneic hematopoietic stem cell transplantation (HSCT)" as used herein means that allogeneic stem cell transplantation is the required therapy for the pediatric ALL patient. In cases, where the pediatric ALL patient is eligible for allogeneic stem cell transplantation, the following two scenarios may be envisaged. First, in one embodiment of the pharmaceutical methods of the invention, the administration of the CD19xCD3 bispecific single chain antibody (alone or preferably as a pharmaceutical composition) can be used to replace allogeneic stem cell transplantation used as a conventional therapy for pediatric ALL patients eligible for transplantation. So the method of the invention can avoid the health risks for the pediatric ALL patients associated with allogeneic HSCT. In addition, about 30% of the transplanted pediatric ALL patients usually relapse after transplantation. So the method of the invention can be used to treat these patients. In an alternative embodiment, the continuous infusion of the CD19xCD3 bispecific single chain antibody to the pediatric ALL patient may be followed by an allogeneic stem cell transplantation. In this embodiment, the administration of a pharmaceutical composition comprising the CD19xCD3 bispecific single chain antibody construct can be used to convert pediatric ALL patients eligible for transplantation into an MRD negative-status before they receive the transplantation. In this way, the method of the invention can be used in order to eliminate MRD, which leads to a lower risk of relapse than the transplantation treatment of MRD-positive patients. The following examples present a pediatric patient (patient 1) who has first been converted into an MRD-negative status upon treatment with the CD19xCD3 bispecific single chain antibody, followed by an allogeneic transplantation. Until now (November 2009), this pediatric patient is still MRD negative.

The term "non-eligible for allogeneic hematopoietic stem cell transplantation (HSCT)" as used herein means those pediatric patients for whom allogeneic stem cell transplantation is not the ALL treatment of choice, for instance, due to medical reasons. It could also happen that no appropriate donor is available for allogeneic stem cell transplantation.

Accordingly, in one embodiment, the acute lymphoblastic leukemia (ALL) is refractory to chemotherapy and/or allogeneic HSCT in pediatric patients non-eligible or no longer eligible for allogeneic HSCT. For example, a pediatric patient may be in such bad clinical condition that no allogeneic stem cell transplantation can be carried out for medical reasons.

Patient 1 shown in the following examples has not been eligible for allogeneic stem cell transplantation before treatment with the CD19xCD3 bispecific single chain antibody, due to his poor state of health. In such a case, treatment with the CD19xCD3 bispecific single chain antibody provides a new therapeutic approach for pediatric ALL.

So far, ALL meant the death sentence for pediatric patients refractory to chemotherapy and non-eligible for allogeneic HSCT. The method of the invention for the first time provides a therapy for this pediatric patient population in that it eliminates the minimal residual disease (MRD) which otherwise would cause a relapse and which would finally kill said patients.

It is within the scope of the pharmaceutical methods of the invention, that the CD19xCD3 bispecific single chain antibody construct be administered to pediatric ALL patients who have received chemotherapy, alone or in combination with an allogeneic stem cell transplantation, and relapsed thereafter.

In another preferred embodiment, the method of the invention is for the treatment, amelioration or elimination of minimal residual disease (MRD) in a pediatric patient with acute lymphoblastic leukemia (ALL).

The term "minimal residual disease (MRD)" as defined herein denotes a term used after treatment e.g. with chemotherapeutics when leukemic cells cannot be found in the bone marrow using standard tests, such as microscopic methods. Rather, more sensitive tests such as flow cytometry (FACS based methods) or polymerase chain reaction (PCR) have to be used in order to find evidence that leukemia cells remained in the bone marrow of the pediatric ALL patient. More specifically, the presence of leukemia cells below the cytological detection limit (5% leukemic cells) is defined as minimal residual disease (MRD). If no MRD is detectable (<10⁻⁴, i.e. less than 1 leukemia cell per 10⁴ bone marrow cells detectable), a complete molecular remission is reached (MRD negativity or MRD negative status). An "MRD positive status" as defined herein means a signal measured by PCR or FACS above detection limit or a quantitative threshold. An "MRD negative status" as defined herein means below detection limit and/or below a quantitiative threshold measured by PCR or FACS. The prognostic value of minimal residual disease quantification in childhood ALL has been described e.g. in Bader et al. (J. Clin. Oncol. 27 (2009): 377-384) or Eckert et al. (Lancet 358 (2001): 1239-41). The MRD status can be measured by PCR or FACS analysis in that the individual cytogenetic abnormalities mentioned herein, and/or rearrangements of immunoglobulin genes or T-cell receptor (TCR) rearrangements are quantitatively detected. For example, PCR analysis can detect fusion transcripts such as bcr/abl or t(4;11) translocations as well as individual clonal rearrangements of immunoglobulins (IgH) and/or T-cell receptor genes (TCR).

As demonstrated in the following examples, upon treatment with the CD19xCD3 bispecific single chain antibody described herein, all acute lymphoblastic leukemia cells could be eliminated from the body of the pediatric ALL patients so that a complete molecular remission (i.e. an MRD negative status) could be achieved.

Recurrent chromosomal abnormalities in the malignant cells of pediatric and adult patients with acute lymphoblastic leukemia are hallmarks of the disease (Harrison and Foroni, Rev. Clin. Exp. Hematol. 6 (2002), 91-113). Specific aberrations which are frequently indicative of consistent underlying molecular lesions can assist or even establish the diagnosis and determine optimal therapy. In childhood ALL, numerous good and high-risk cytogenetic subgroups have been identified which are regularly used to stratify patients to particular therapies (Pui and Evans, N. Engl. J. Med. 354 (2006), 166-178). In adult ALL, the role of cytogenetics in patient management has largely been centered on the presence of the Philadelphia (Ph) chromosome which usually arises from t(9;22)(q34;q11.2) and results in BCR-ABL fusion (Faderl et al., Blood 91 (1998), 3995-4019). Although the overall incidence of Ph+ ALL in adults is approximately 25%, it is correlated with age and rises to greater than 50% among patients older than the age of 55 years (Appelbaum, American Society of Clinical Oncology 2005 education book. Alexandria: ASCO, 2005: 528-532). Other cytogenetic translocations associated with specific molecular genetic abnormalities in acute lymphoblastic leukemia (ALL) are shown in Table 1 and also described in Schultz et al. or Bader et al., loc. cit.

**Table 1:**

| **Cytogenetic translocation** | **Molecular genetic abnormality** |
|---|---|
| t(9;22)(q34;q11) | BCR-ABL fusion(P185) |
| t(12;21)CRYPTIC | TEL-AML1 fusion |
| t(1;19)(q23;p13) | E2A-PBX fusion |
| t(4;11)(q21;q23) | MLL-AF4 fusion |
| t(8;14)(q24;q32) | IGH-MYC fusion |
| t(11;14)(p13;q11) | TCR-RBTN2 fusion |

Cytogenetics, has been increasingly recognized as an important predictor of outcome in ALL (Moormann et al., Blood 109 (2007), 3189-97).

Some cytogenetic subtypes have a worse prognosis than others. These include e.g.:
(i) A translocation between chromosomes 9 and 22, the Philadelphia chromosome (Ph+), occurs in about 20% of adult and 5% in pediatric cases of ALL.
(ii) A translocation between chromosomes 4 and 11 occurs in about 4% of cases and is most common in infants under 12 months.

Rearrangements of immunoglobulin genes or T-cell receptor (TCR) rearrangements and their role in ALL have been described in the art (see e.g. Szczepański et al., Leukemia 12 (1998), 1081-1088).

In another preferred embodiment of the pharmaceutical methods of the invention, said pediatric ALL patient is MRD-positive in complete hematological remission.

The term "remission" or "complete hematological remission" as used herein is to be understood as having no evidence of ALL disease after standard treatment, e.g. after chemotherapy and/or transplantation. This means that the bone marrow contains fewer than 5% blast cells as determined by light microscopy, the blood cell counts are within normal limits, and there are no signs or symptoms of the ALL disease. Nevertheless, it may occur that not all leukemia cells could be eliminated from the body. Such a patient, though staged as being in remission or complete hematological remission, is still MRD positive. These remaining tumor cells may give rise to recurrent leukemia. The pharmaceutical methods of the invention can be used to kill these remaining tumor cells in order to prevent recurrence of the leukemia originating from the occult leukemia cells remaining in the body after primary therapy. In this way, the pharmaceutical means and methods help to prevent disease relapse in pediatric ALL patients.

In contrast, a "molecular complete remission" means that there is no evidence of leukemia cells in biopsies of the bone marrow, even when using very sensitive tests such as PCR or FACS analysis. Put in other words: If no MRD is detectable (<10⁻⁴, i.e. less than one leukemia cell per 10⁴ bone marrow cells), a complete molecular remission is reached.

In another preferred embodiment of the pharmaceutical methods of the invention, the administration of said pharmaceutical composition converts MRD-positive pediatric acute lymphoblastic leukemia (ALL) into an MRD negative status.

In another preferred embodiment of the pharmaceutical methods of the invention, MRD is measured with quantitative detection of at least one of the cytogenetic abnormalities or rearrangements selected from the group consisting of:
t(12;21)[TEL-AML1];
t(1;19;)[E2A-PBX];
t(4;11)[AF4-MLL];
t(9;22)[BCR-ABL];
hyperdiploidy or simultaneous trisomies of chromosomes 4, 10, and 17;
hypodiploidy (i.e. less than 44 chromosomes);
rearrangements of immunoglobulin genes; and
T-cell receptor (TCR) rearrangements.

MRD is measured with quantitative detection of at least one of: (i) the individual cytogenetic abnormalities mentioned herein, such as t(12;21)(TEL-AML1]; t(1;19;)[E2A-PBX]; t(4;11)[AF4-MLL]; t(9;22)[BCR-ABL]; hyperdiploidy (or simultaneous trisomies of chromosomes 4, 10, and 17), hypodiploidy (i.e. less than 44 chromosomes), or (ii) the rearrangements of immunoglobulin genes or T-cell receptor (TCR) rearrangements; see also Table 1. Said quantitative detection of the mentioned cytogenetic abnormalities or rearrangements are preferably carried out by using, for example, PCR or FACS analysis.

The method of the invention provides a therapeutic approach for the treatment, amelioration or elimination of MRD, thereby reducing or even abolishing the risk of relapse for the pediatric ALL patient. Notably, curative treatment of MRD in pediatric ALL patients has not yet been available until now.

In another preferred embodiment of the pharmaceutical methods of the invention, said pediatric patient having MRD shows a signal for a cytogenetic abnormality above detection limit and/or at least one marker by rearrangement with a sensitivity of ≥10⁻⁴. Preferably, MRD is detected by PCR and/or FACS analysis.

The individual cytogenetic abnormalities mentioned herein include e.g. t(12;21)[TEL-AML1]; t(1;19;)[E2A-PBX]; t(4;11)[AF4-MLL]; t(9;22)[BCR-ABL]; hyperdiploidy (or trisomy of chromosomes 4, 10, and 17), hypodiploidy. The rearrangements include, for instance, rearrangements of immunoglobulin genes or T-cell receptor (TCR) rearrangements; see also Table 1. The MRD represented by ALL cells carrying said cytogenetic abnormalities and/or rearrangements can be detected by PCR or FACS analysis.

For example, the term "above bcr/abl signal above detection limit" as used herein means that PCR or FACS analysis leads to a detectable bcr/abl signal. Similary, a t(4;11) translocation signal means that said translocation can be detected by PCR or FACS. This applies mutatis mutandis to the other cytogenetic abnormalities or rearrangements mentioned herein. The term "with a sensitivity of ≥10⁻⁴ " in context with rearrangements means that at least one leukemia cell or more than one leukemia cells (with the mentioned TCR or immunoglobulin rearrangement) per 10⁴ bone marrow cells can be detected by the mentioned methods.

In another preferred embodiment of the pharmaceutical methods of the invention, the time to molecular relapse (detectable by the assays described above) is more than 6 months, preferably more than 7, 8, 9, 10, 11 or 12 months, or even more preferred for 2, 3, 4, 5 or more years.

The term "molecular relapse" as used herein means that said pediatric patient shows a signal for a cytogenetic abnormality above detection limit and/or at least one marker by rearrangement with a sensitivity of ≥10⁻⁴, by PCR and/or FACS, as set forth above.

In another preferred embodiment of the pharmaceutical methods of the invention, the corresponding variable heavy chain regions (V_{H}) and the corresponding variable light chain regions (V_{L}) regions in said CD19xCD3 bispecific single chain antibody construct are arranged, from N-terminus to C-terminus, in the order, V_{L}(CD19)-V_{H}(CD19)-V_{H}(CD3)-V_{L}(CD3).

The corresponding variable heavy chain regions (V_{H}) and the corresponding variable light chain regions (V_{L}) regions of the CD3 and CD19 binding domains of the CD19xCD3 bispecific single chain antibody are shown in SEQ ID NOs. 3 to 10, respectively. The corresponding CDR regions of the respective VH and VL regions of the mentioned CD19xCD3 bispecific single chain antibody are shown in SEQ ID NOs. 11 to 22.

In another preferred embodiment of the pharmaceutical methods of the invention, said CD19xCD3 bispecific single chain antibody construct comprises an amino acid sequence as set forth in SEQ ID NO. 1, or an amino acid sequence at least 90%, preferably at least 95% identical to SEQ ID NO. 1.

The invention describes a CD19xCD3 bispecific single chain antibody molecule comprising an amino acid sequence as depicted in SEQ ID NO. 1, as well as an amino acid sequence at least 90 % or preferably 95 % identical, most preferred at least 96, 97, 98, or 99 % identical to the amino acid sequence of SEQ ID NO. 1. The invention describes also the corresponding nucleic acid sequence as depicted in SEQ ID NO. 2 as well as a nucleic acid sequence at least 90 %, preferably 95 % identical, most preferred at least 96, 97, 98, or 99 % identical to the nucleic acid sequence shown in SEQ ID NO. 2. It is to be understood that the sequence identity is determined over the entire nucleotide or amino acid sequence. Moreover, it is to be understood that a bispecific single chain antibody molecule comprising an amino acid sequence at least 90 % or preferably 95 % identical, most preferred at least 96, 97, 98, or 99 % identical to the amino acid sequence of SEQ ID NO. 1 contains all of the CDR sequences shown in SEQ ID NOs. 11 to 22. For sequence alignments, for example, the programs Gap or BestFit can be used (Needleman and Wunsch J. Mol. Biol. 48 (1970), 443-453; Smith and Waterman, Adv. Appl. Math 2 (1981), 482-489), which is contained in the GCG software package (Genetics Computer Group, 575 Science Drive, Madison, Wisconsin, USA 53711 (1991). It is a routine method for those skilled in the art to determine and identify a nucleotide or amino acid sequence having e.g. 90%, 95%, 96%, 97%, 98% or 99% sequence identity to the nucleotide or amino acid sequences of the CD19xCD3 bispecific single single chain antibody described herein. For example, according to Crick's Wobble hypothesis, the 5' base on the anti-codon is not as spatially confined as the other two bases, and could thus have non-standard base pairing. Put in other words: the third position in a codon triplet may vary so that two triplets which differ in this third position may encode the same amino acid residue. Said hypothesis is well known to the person skilled in the art (see e.g. http://en.wikipedia.org/wiki/Wobble_Hypothesis; Crick, J Mol Biol 19 (1966): 548-55). It is furthermore a routine procedure for those skilled in the art to determine cytotoxic activity of such an amino acid sequence having e.g. 90%, 95%, 96%, 97%, 98% or 99% sequence identity to the nucleotide or amino acid sequences of the CD19xCD3 bispecific single single chain antibody described herein. Cytotoxic activity of the CD19xCD3 bispecific single chain antibody or an antibody construct having e.g. 90%, 95%, 96%, 97%, 98% or 99% sequence identity to the amino acid sequences of the CD19xCD3 bispecific single single chain antibody can be determined by methods as illustrated e.g. in WO 99/54440, WO 2004/106381, or WO 2007/068354.

In another preferred embodiment of the pharmaceutical methods of the invention, the pharmaceutical composition comprising a CD19xCD3 bispecific single chain antibody construct is to be administered by continuous infusion for at least four weeks followed by a 2-week treatment-free interval. One treatment cycle is to be understood as continuous infusion of said antibody for at least four weeks, followed by a 2-week treatment-free interval. This interval may in turn be followed by one or more treatment cycles or by an allogeneic HSCT. Preferably, said administration by continuous infusion of the CD19xCD3 bispecific single chain antibody (i.e. one treatment cycle) is to be repeated at least two, three times, four, five, six, seven, eight, nine or even up to ten times, after determination of a MRD negative status, for consolidation.

In one embodiment of the pharmaceutical methods of the invention, the method is prior to allogeneic stem cell transplantation to convert the MRD positive ALL into an MRD negative status.

In another embodiment of the pharmaceutical methods of the invention, the method is after allogeneic HSCT, for example, in cases where the pediatric ALL relapsed after conventional ALL therapy using chemotherapy and/or allogeneic stem cell transplantation.

In another preferred embodiment of the pharmaceutical methods of the invention, the CD19xCD3 bispecific single chain antibody construct is to be administered in a daily dose of 10µg to 100µg per square meter patient body surface area.

As used herein, a dose range which is defined as "X to Y" equates with a dose range which is defined as "between X and Y". The range includes the upper limit and also the lower limit. This means that for example a daily dose of 10µg to 100µg per square meter patient body surface area includes "10µg" and "100µg".

In an even more preferred embodiment of the pharmaceutical methods of the invention, the CD19xCD3 bispecific single chain antibody construct is to be administered in a daily dose of 15µg, 30µg, 60 µg or 90 µg per square meter patient body surface area. Even more preferred, said antibody is to be administered in a daily dose of 15 to 30 µg per square meter patient body surface area, most preferred in a daily dose of 15 or 30 µg per square meter patient body surface area.

The average body surface area of a pediatric patient is hereby calculated in the context of the method or use according to the invention to be in a range of about 0,2 to 2,2 m². For the calculation see e.g. the formulas provided by http://www.cato.eu/koerperoberflaeche-kinder.html

In another embodiment of the pharmaceutical methods of the invention, the pediatric acute lymphoblastic leukemia (ALL) is newly diagnosed, refractory or relapsed pediatric ALL, or refractory and relapsed ALL. Newly diagnosed pediatric ALL as used herein means that the ALL disease has been diagnosed in the pediatric patient for the first time.

In another embodiment of the pharmaceutical methods of the invention, said method is for a patient with high risk of relapse according to the COGAALL03B1 classification of acute lymphoblastic leukemia.

Advantageously, the pharmaceutical composition comprising the CD19xCD3 bispecific single chain antibody as described herein further comprises, optionally (a) reaction buffer(s), storage solutions and/or remaining reagents or materials required for the recited method or use. Furthermore, said components can be packaged individually in vials or bottles or in combination in containers or multicontainer units.

In order to evaluate safety and tolerability of the CD19xCD3 bispecific single chain antibody as described herein, the compound is to be administered by long-term continuous infusion.

It has been found that the beneficial and unexpected effects of the pharmaceutical methods of the invention can be obtained by administering the CD19xCD3 bispecific single chain antibody in a daily dose of 10 microgram to 100 microgram per square meter body surface area. The daily dose may be kept constant over the administration period. However, it is also within the ambit of this embodiment that for the initial day(s) of the infusion period a lower dose of CD19xCD3 bispecific single chain antibody be administered ("initial dose") prior to the pharmaceutical methods described herein, whereas for the remaining infusion period a higher dose ("maintenance dose") be applied. This measure may help to adapt the patient's body to the antibody treatment and/or to avoid undesired side effects. For example, 5 microgram of bispecific single chain antibody per square meter body surface area may be administered as an initial dose at the first day(s) (e.g. first day, or first and second day, or first, second and third day, etc. up to the seventh day) of the infusion period followed by administration of 15 microgram per square meter body surface as daily dose for the remaining period. Or 5 microgram of bispecific single chain antibody per square meter body surface area may be administered as initial dose at the first day(s) of the infusion period followed by administration of 30 or 45 microgram per square meter body surface as daily dose for the remaining period. It is also envisaged that 5 microgram of CD19xCD3 bispecific single chain antibody per square meter body surface area may be administered at the first day(s) of the infusion period, followed by administration of 15 microgram of CD19xCD3 bispecific single chain antibody per square meter body surface area for the following day(s) of the infusion period, followed by administration of 30 or 45 microgram per square meter body surface as daily (maintenance) dose for the remaining administration period of a total of at least 4 weeks. The two initial doses may be administered not only for one day, but also for 2, 3, 4, 5, 6 or 7 days or even longer. The average body surface area of a patient is hereby calculated in the context of the pharmaceutical methods according to the invention to be in a range of 0,2 to 2,2 m².

The uninterrupted administration of the CD19xCD3 bispecific single chain antibody may be intravenous, parenteral, subcutaneous, transdermal, intraperitoneal, intramuscular or pulmonary. The intravenous mode of administration will in most cases be the mode of choice for uninterruptedly administering the CD19xCD3 bispecific single chain antibody and, as the case may be, for co-administration of a pharmaceutical agent as part of a regimen of co-therapy. As such, intraveneous administration is especially preferred. In this case, a suitable metering device such as the multi-therapy infusion pump model 6060 manufactured by Baxter may advantageously be chosen. Whatever metering device is chosen, it should be of such design and construction as to minimize or, better, preclude an interruption of administration of therapeutic agent in the event of cartridge exchange and/or power cell replacement or recharging. This may be accomplished, for example by choosing a device with a secondary reservoir of CD19xCD3 bispecific single chain antibody solution apart from the cartridge to be exchanged so that continuous infusion from this secondary reservoir into the pediatric patient may continue even while the empty or almost empty cartridge is removed and replaced with a fresh one.

A mode of intraveneous administration and, as the case may be, of co-administration as part of a regimen of co-therapy involves the implantation of a pump into the body of the pediatric patient for metering such administration. One of ordinary skill in the art is aware of such metering pumps, for example model 6060 manufactured by Baxter as set forth above.

As a non-limiting example, it may be that the uninterrupted, i.e. continuous administration is to be realized by a small pump system worn by or implanted into the pediatric patient for metering the influx of therapeutic agent into the body of the patient. Such pump systems are generally known in the art, and commonly rely on periodic exchange of cartridges containing the therapeutic agent to be infused. When exchanging the cartridge in such a pump system, a temporary interruption of the otherwise uninterrupted flow of therapeutic agent into the body of the pediatric patient may ensue. In such a case, the phase of administration prior to cartridge replacement and the phase of administration following cartridge replacement would still be considered within the meaning of the pharmaceutical methods of the invention to together make up one "uninterrupted administration" of such therapeutic agent. The same would apply for very long administrations in which the cartridge would require replacement more than once, or in which the power cells driving the pump would require replacement, leading to a temporary offset of the flow of therapeutic solution into the body of the patient.

Appropriate measures should also be taken to minimize the danger of infection at the puncture site of administration into the patient's body, as such long-term wounds are especially prone to such infection. The above also applies for intramuscular administration via a similar delivery system.

The continuous administration may be transdermal by way of a patch worn on the skin and replaced at intervals. One of skill in the art is aware of patch systems for drug delivery suitable for this purpose. It is of note that transdermal administration is especially amenable to uninterrupted administration, as exchange of a first exhausted patch can advantageously be accomplished simultaneously with the placement of a new, second patch, for example on the surface of the skin immediately adjacent to the first exhausted patch and immediately prior to removal of the first exhausted patch. Issues of flow interruption or power cell failure do not arise.

Furthermore, the invention relates to a CD19xCD3 bispecific single chain antibody construct for use in a method of treatment, amelioration or elimination of pediatric acute lymphoblastic leukemia (ALL), wherein said CD19xCD3 bispecific single chain antibody is to be prepared for administration to a pediatric patient.

The invention further relates to the use of a CD19xCD3 bispecific single chain antibody construct for the preparation of a pharmaceutical composition for the treatment, amelioration or elimination of pediatric acute lymphoblastic leukemia (ALL). Therefore, the present invention also relates to a CD19xCD3 bispecific single chain antibody construct as defined herein for the treatment, amelioration or elimination of pediatric acute lymphoblastic leukemia (ALL). The embodiments disclosed herein in context of the pharmaceutical methods apply here mutatis mutandis for the preparation of the corresponding pharmaceutical composition comprising the single chain constructs anti CD19xCD3 constructs for the administration to a pediatric patient, in particular in the treatment, amelioration or elimination of pediatric acute lymphoblastic leukemia (ALL).

Preferably, said pediatric acute lymphoblastic leukemia (ALL) is pediatric B-lineage acute lymphoblastic leukemia (ALL), preferably pediatric B-precursor acute lymphoblastic leukemia ALL, more preferably pediatric pro-B ALL, pre-B ALL or common ALL (cALL). Even more preferred, the ALL is cALL.

In a preferred embodiment of the mentioned medical uses, said B-precursor ALL is relapsed and/or refractory to conventional ALL treatment, such as chemotherapy and/or HSCT.

Preferably, said acute lymphoblastic leukemia (ALL) relapses within about three years of diagnosis.

In another preferred embodiment of the mentioned medical uses, the CD19xCD3 bispecific single chain antibody construct is for the treatment, amelioration or elimination of minimal residual disease (MRD) in a pediatric patient with acute lymphoblastic leukemia (ALL). Preferably, said pediatric patient is MRD-positive in complete hematological remission.

In a further preferred embodiment of the mentioned medical uses, the administration of said CD19xCD3 bispecific single chain antibody converts MRD positive pediatric acute lymphoblastic leukemia (ALL) into an MRD negative status.

Preferably, MRD is measured with quantitative detection of individual cytogenetic abnormalities or rearrangements, as defined herein, using PCR and/or FACS analysis. Even more preferred, the pediatric ALL patient shows a signal for a cytogenetic abnormality above detection limit and/or at least one marker by rearrangement with a sensitivity of ≥10⁻⁴.

In another preferred embodiment of the mentioned medical uses, the time to molecular relapse detectable by the indicated detection methods is more than 6 months.

In another preferred embodiment of the mentioned medical uses, the corresponding variable heavy chain regions (V_{H}) and the corresponding variable light chain regions (V_{L}) regions in said CD19xCD3 bispecific single chain antibody construct are arranged, from N-terminus to C-terminus, in the order, V_{L}(CD19)-V_{H}(CD19)-V_{H}(CD3)-V_{L}(CD3). Preferably, said CD19xCD3 bispecific single chain antibody construct comprises an amino acid sequence as set forth in SEQ ID NO. 1, or an amino acid sequence at least 90%, preferably 95% identical to SEQ ID NO. 1.

In a further preferred embodiment of the mentioned medical uses, one treatment cycle is a continuous infusion for at least four weeks, followed by a 2-week treatment-free interval.

Preferably, said administration is to be repeated at least two, three, four, five, six, seven, eight, nine of ten times, after determination of a MRD negative status.

In another embodiment, the administration is prior to allogeneic stem cell transplantation to convert the MRD positive ALL into an MRD negative status.

In an alternative embodiment, the administration is after allogeneic stem cell transplantation.

In another preferred embodiment of the mentioned medical uses, the CD19xCD3 bispecific single chain antibody construct is to be administered in a daily dose of 10µg to 100µg per square meter patient body surface area.

Preferably, the CD19xCD3 bispecific single chain antibody construct is to be administered in a daily dose of 15µg to 30µg per square meter patient body surface area.

In another embodiment of the mentioned medical uses, the administration of the CD19xCD3 bispecific single chain antibody construct replaces allogeneic stem cell transplantation in pediatric patients eligible for allogeneic stem cell transplantation.

The embodiments, definitions and explanations provided with respect to the pharmaceutical methods of the invention apply mutatis mutandis to the medical uses of the CD19xCD3 bispecific single chain antibody construct described herein.

The Figures show:

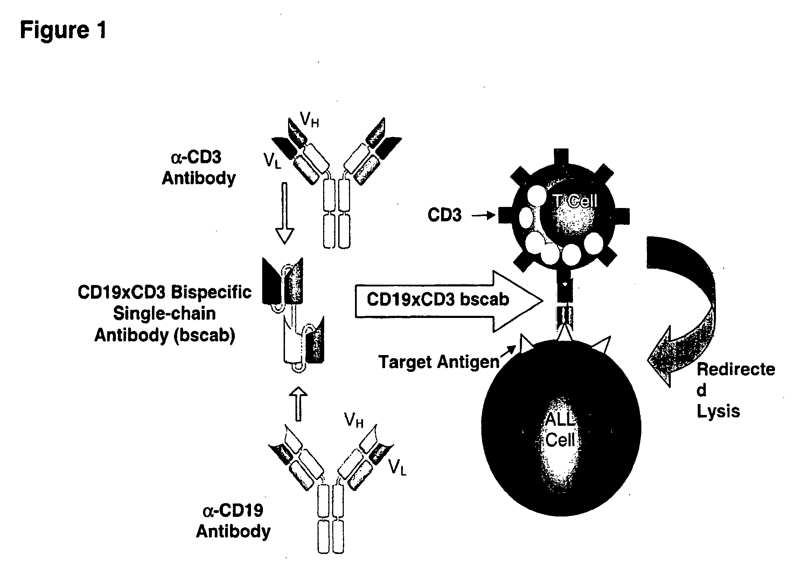

**Figure 1****:** CD19xCD3 bispecific single chain antibody (CD19xCD3 bscab) mode of action. CD19xCD3 bispecific single chain antibody (SEQ ID NO. 1) redirects CD3-positive cytotoxic T cells to eliminate human acute lymphoblastic leukemia cells carrying the CD19 antigen.

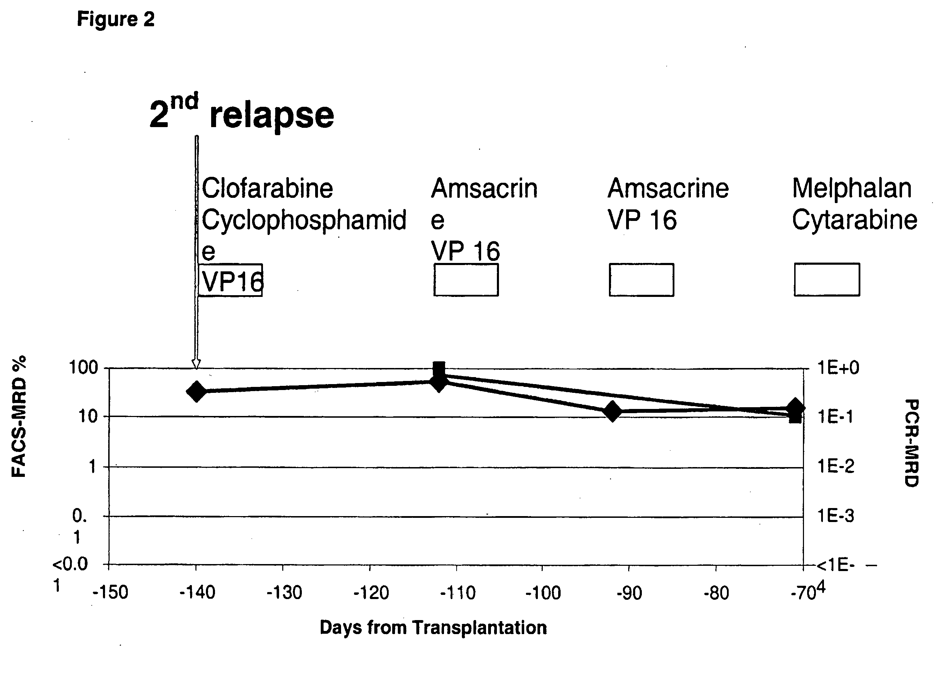

**Figure 2****:** Failure of conventional ALL therapy in a 7-year old patient with common ALL. One year after HSCT, the pediatric patient experienced a second bone marrow relapse and received subsequent chemotherapy including 1 cycle of Clofarabine/Cyclophosphamide/VP16, 2 cycles of Amsacrine, VP16, prednisone and 1 cycle of Melphalan/Cytarabine. Despite this aggressive chemotherapy, the patient had persistant morphologic disease throughout treatment, as demonstrated by the high MRD levels. Left y-axis indicates FACS-MRD %, right y-axis indicates PCR-MRD (from 10⁰ to <10⁻⁴), x-axis indicates days from first HSCT; see Example 4.

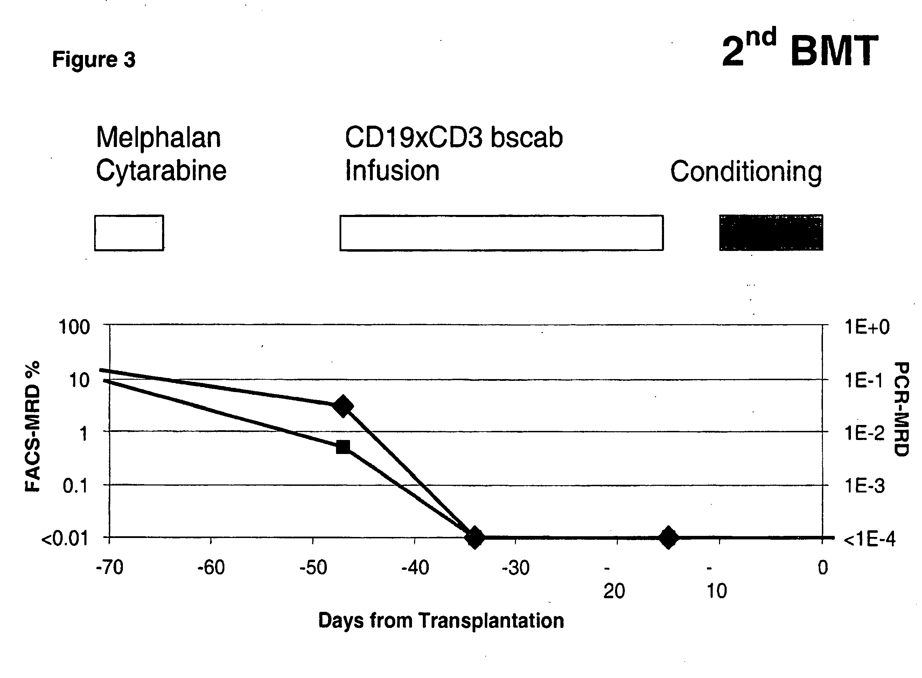

**Figure 3****:** Successful treatment of the pediatric patient by administration of CD19xCD3 bispecific single chain antibody (CD19xCD3 bscab; SEQ ID NO. 1), as demonstrated by MRD prior, during and after treatment with said antibody. After failure of conventional ALL therapy, the patient was treated with a continuous infusion of 15 µg/m² CD19xCD3 bispecific single chain antibody (SEQ ID NO. 1) for 5 weeks. Upon antibody treatment, MRD negativity could be reached. In 10/2008, i.e. 2 weeks after end of treatment with the CD19xCD3 bispecific single chain antibody (day 0 in Figures 3 to 5), he underwent a second HSCT from his haploidentical mother using a non-myeloablative preparative regimen consisting of Clofarabine, Thiotepa and melphalan (Lang) indicated by the conditioning bar. Left y-axis indicates FACS-MRD %, right y-axis indicates PCR-MRD (from 10° to <10⁻⁴), x-axis indicates days from first HSCT, day "0" indicates the day of the second HSCT (following the antibody treatment); see Example 4.

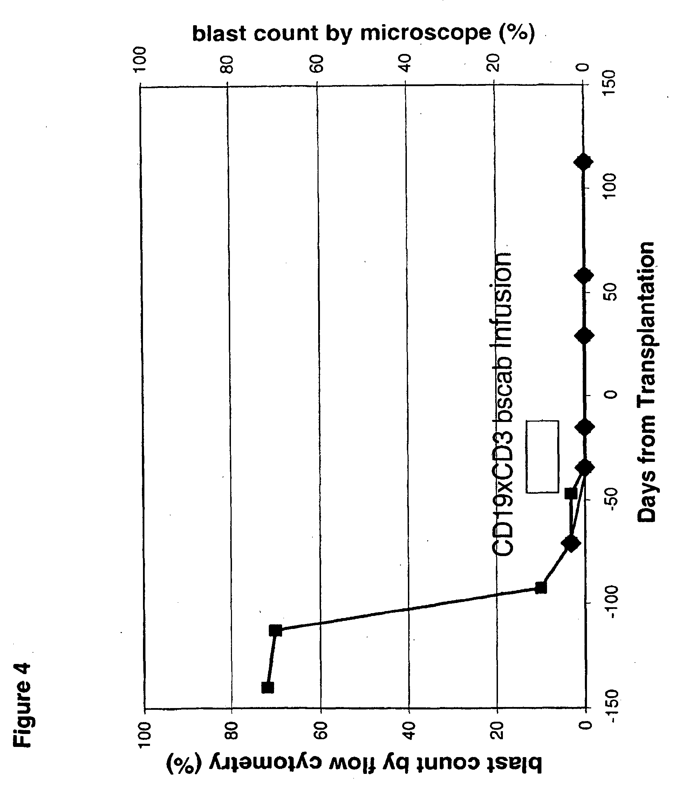

**Figure 4****:** Temporal development of blast counts before and after treatment with the CD19xCD3 bispecific single chain antibody (CD19xCD3 bscab; SEQ ID NO. 1). A bone marrow analysis at day 10 of CD19xCD3 bispecific single chain antibody treatment revealed a complete elimination of the bone marrow blasts (MRD < 10⁻⁴) by the antibody. Another BM analysis at the end of CD19xCD3 bispecific single chain antibody treatment at day 35 confirmed complete absence of leukemic blasts from the bone marrow (MRD <10⁻⁴). Left y-axis indicates blast count by flow cytometry in %, right y-axis indicates blast count by microscopy in %, x-axis indicates days from HSCT, with negative days indicating days from first HSCT, day "0" indicating the day of the second HSCT (following the antibody treatment) and positive days indicating days after the second HSCT.

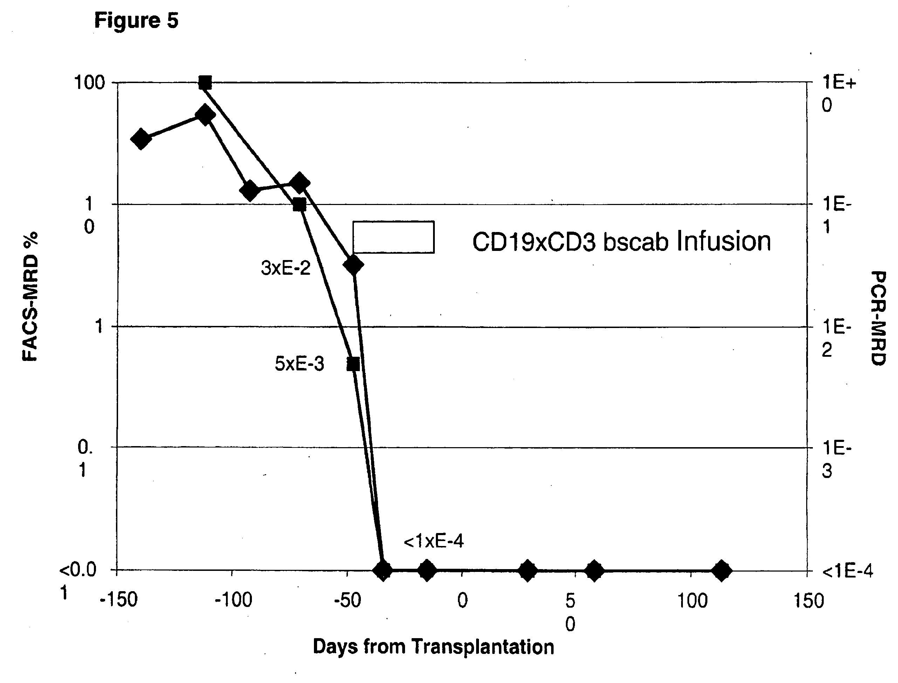

**Figure 5****:** Decrease of MRD levels upon treatment with CD19xCD3 bispecific single chain antibody (CD19xCD3 bscab; SEQ ID NO. 1). Upon antibody treatment, MRD negativity could be reached. As of 6/2009, the patient remains in MRD-negative complete molecular remission. Left y-axis indicates FACS-MRD %, right y-axis indicates PCR-MRD (from 10° to <10⁻⁴), x-axis indicates days from HSCT, with negative days indicating days from first HSCT and "0" indicating day of second HSCT and positive days indicating days from second HSCT.

The invention is further illustrated by the following examples:

### Examples:

### 1. CD19xCD3 bispecific single chain antibody

The generation, expression and cytotoxic activity of the CD19xCD3 bispecific single chain antibody has been described in WO 99/54440. The corresponding amino and nucleic acid sequences of the CD19xCD3 bispecific single chain antibody are shown in SEQ ID NOs. 1 and 2, respectively. The VH and VL regions of the CD3 binding domain of the CD19xCD3 bispecific single chain antibody are shown in SEQ ID NOs. 7 to 10, respectively, whereas the VH and VL regions of the CD19 binding domain of the CD19xCD3 bispecific single chain antibody are shown in SEQ ID NOs 3 to 6, respectively.

### 2. Phenotypic analysis of lymphocytes and chimerism analysis

For the phenotypic analysis of lymphocytes and chimerism analysis, the patient's blood samples were collected prior, during and after treatment with CD19xCD3 bispecific single chain antibody using EDTA-containing collection tubes. Absolute number of lymphocytes in the blood samples were determined through differential blood analysis on a Advia. Lymphocytes were stained using fluorescence-labeled antibodies directed against CD3, CD4, CD8, CD19 and CD56 (all obtained from Becton-Dickinson, Heidelberg, Germany). The analysis of labelled cells and data collection were performed with a FACSCalibur (Becton-Dickinson).

### 3. Detection of MRD

For the detection of MRD, either a polymerase chain reaction (PCR)-based assay (Bader et al., loc. cit.) or FACS analysis was used. Briefly, DNA was isolated by DNeasy Blood&Tissue Kit (Qiagen GmbH, Hilden, Germany). Immunoglobulin and T-cell receptor gene rearrangement as PCR-based targets have recently been shown to be stable markers for monitoring MRD in acute lymphoblastic leukemia after stem cell transplantation (Kreyenberg, Leukemia, 2009).

### 4. Case reports

### 4.1 Case report patient 1

This 7-year old patient was diagnosed with high risk CD10+ common ALL (CD19-, CD34-positive; CD45 reduced; TCR rearrangement; CNS negative) in 2004. After treatment, he experienced a bone marrow relapse in June 2006 and was treated according to the ALL-REZ BFM study in the S3 arm. After two chemotherapy cycles, the patient had persistant disease and achieved a complete remission after 3 courses of clofarabine. In 2007, he received an allogeneic HSCT from a 9 out of ten HLA-allele matched unrelated donor after conditioning with total body irradiation and Etoposide. One year after HSCT, he experienced another bone marrow relapse and received subsequent chemotherapy including 1 cycle of Clofarabine/Cyclophosphamide/VP16 ( Nobuko), 2 cycles of Amsacrine, VP16, prednisone (Hamburg) and 1 cycle of Melphalan/Cytarabine; see Figure 2. Despite this aggressive chemotherapy, the patient had persistant morphologic disease throughout treatment, as evident from the high MRD levels in Figure 2. He was then treated under compassionate use with a continuous infusion of 15 µg/m² of the CD19xCD3 bispecific single chain antibody (blinatumomab; SEQ ID NO. 1) for 5 weeks; see Figure 3. Serial analysis of the lymphocyte populations prior (day 0) and during treatment with the mentioned antibody showed an impressive expansion of CD8+ T-lymphocytes, whereas no change in CD4+ T-cells and CD56+ NK cells was noted. Upon antibody treatment, MRD negativity could be achieved. Concomitant chimerism analyis showed 100% donor chimerism. As shown in Figure 4, bone marrow analysis at the 10^{th} day of CD19xCD3 bispecific single chain antibody treatment revealed a complete elimination of the bone marrow blasts (MRD < 10⁻⁴). Another BM analysis at the end of CD19xCD3 bispecific single chain antibody treatment showed again complete absence of leukemic blasts from the bone marrow (MRD <10⁻⁴). The serial analysis of MRD prior, during and after treatment with said antibody is depicted in Figure 5. Before antibody treatment, the pediatric patient had a very poor prognosis and was not eligible to HSCT due to his bad clinical condition. Upon antibody treatment, MRD negativity could be achieved. Besides from transient discrete signs of ataxia, no major side effects were observed and no signs of GvHD were seen despite the impressive expansion of donor-derived CD8+ T-lymphocytes. In 10/2008, 2 weeks after end of treatment with CD19xCD3 bispecific single chain antibody (i.e. day 0 in Figures 3 to 5), he underwent a second HSCT from his haploidentical mother using a nonmyeloablative preparative regimen consisting of Clofarabine, Thiotepa and melphalan (Lang). As of 11/2009, the patient has remained in MRD-negative complete remission.

### 4.2 Case report Patient 2

This fifteen-year old patient was diagnosed with Philadelphia chromosome- and CD19-positive B-precursor ALL in April 2001. After chemotherapy, he received a HSCT from a HLA-identical sibling in October 2001 after conditioning with TBI of 12 Gy and etoposide. In 2002, a bone marrow relapsed was diagnosed and another remission was achieved with matinib and chemotherapy. He then received a second HSCT from an HLA-identical unrelated donor in October 2004. In March 2008, a second relapse was diagnosed and he was treated with low-dose chemotherapy and Dasatinib due to resistance to Imatinib. After additional chemotherapy with Clofarabin and Cytosin/Arabinosid, he achieved a molecular remission and received a third allogeneic HSCT from his 3 out of 6 HLA-allele-mismatched haploidentical father with post-transplant treatment with Dasatinib. Due to gastrointestinal bleeding and dilatative cardiomyopathy, Dasatinib was discontinued 5 months after transplantation. In April 2009, a combined central nervous system (CNS) relapse with 7x10⁹/L blasts in the CNS and 3% blasts in the bone marrow was diagnosed. The patient was then treated with Nilotinib, intrathecal chemotherapy and fractionated irradiation of the CNS with 18 Gy. Three months after this treatment, the patients' bone marrow remained MRD-positive at a level of 1.1 x10⁻³ while the CNS was free of blasts. Chimerism analysis of peripheral blood revealed a complete donor-derived hematopoiesis from his haploidentical father.

The patient was then treated under compassionate use with single-agent blinatumomab at 15 µg/m²/day for 4 weeks by continuous infusion without any side effects. A bone marrow aspiration at the end of the treatment showed complete remission with undetectable MRD in the bone marrow below <1x10⁻⁴. As patient 1, patient 2 did not show any signs of GvHD during or after treatment with blinatumomab. Two weeks after end of the treatment with blinatumomab, the patient experienced a transient haemolytic reaction triggered by an infection. Patient 2 is currently without relapse four weeks after treatment. He is well and attending school.

### 4.3 Summary

The drugs so far available for treatment of ALL are of low specificity and affect a variety of other cells resulting in severe side effects such as immunosuppression, bone marrow aplasia, mucositis, neuropathy, cardiotoxicity, and alopecia. Better targeted approaches with fewer side effects are therefore urgently needed. Furthermore, in some patients, ALL clones develop complete resistance to conventional chemotherapy, in particular those clones arising post HSCT, such that drugs with a distinct mode of action are desirable. The efficacy of antibody-dependent cytotoxicity via the Fc receptors of NK cells and granulocytes in combination with chemotherapy could be demonstrated in pediatric ALL using monoclonal chimeric antibody rituximab. Currently, blinatumomab is the only antibody in clinical trials allowing the engagement of cytotoxic T cells for targeting CD19 in B-cell non-Hodgkin lymhomas and lymphatic leukemias. T cells are considered to have a higher cytotoxic potential than those immune cells engaged by conventional monoclonal antibodies.

The allogeneic GvL effect is supposed to be one of the main reasons for the anti-leukemic efficacy of HSCT. Unfortunately, the occurrence of GvL is often associated with GvHD, which is still a major cause of morbidity and mortality after allogeneic HSCT. Therefore, the induction of GvL in the absence of GvHD is a topic of intensive research. One approach for the induction of a GvL effect is donor lymphocyte infusion (DLI). While DLI is very effective in the treatment of CML, it is less effective in the post-transplant treatment of relapsed ALL and often leads to the occurrence of chronic GvHD with significant impairment of quality of life for the affected children.

One possible approach to induce GvL without GvHD is the in-vivo activation of donor-derived T lymphocytes after HSCT using low doses of the T-cell engaging antibody blinatumomab, which can direct T-lymphocytes against the patients' CD19-positive ALL blasts. This antibody has shown impressive anti-lymphoma and anti-leukemic activity in the autologous situation, but has never been tested in relapsed ALL after HSCT or in children. In the autologous situation, molecular remissions have been induced in 13 out of 16 evaluable adult patients with MRD-positive ALL. Both pediatric patients described above showed an impressive anti-leukemic response after initiation of treatment. In both cases, MRD dropped below the level of detection without any signs of GvHD, despite an extensive expansion of donor-derived T-cells. This could be due to the fact that the action of blinatumomab is independent from regular peptide antigen presentation and may not involve engagement of the naïve T cell repertoire.

Dose levels of blinatumomab as low as 15 µg/m² per day were sufficient to induce an expansion of donor-derived T-lymphocytes and to eliminate the ALL blasts below the detection level of MRD. This highlights that T-cell engagement is very different from the mode of action of conventional monoclonal antibodies, which require much higher doses and cannot engage cytotoxic T-cells due to their lack of Fc receptors.

Blinatumomab was well tolerated in both patients causing only fatigue, mild ataxia and tremor of CTCAE grade 1-2. In patient 2, no side effects were seen at all despite his intensive pre-treatments. The continuous i.v. infusion over several weeks was well accepted by both pediatric patients.

From this first clinical experience in pediatric ALL, the inventors conclude that blinatumomab is well tolerated and can rapidly induce complete haematological and MRD-negative remissions in children with therapy-refractory disease after multiple relapses of CD19-positive B-precursor ALL post-allogeneic HSCT. It is noteworthy that none of the patients showed any signs of GvHD despite an expansion of donor-derived T-lymphocytes, even after mismatched haploidentical transplantation.

These first results indicate that treatment of pediatric acute lymphoblastic leukemia (ALL) patients with the CD19xCD3 bispecific single chain antibody is able to completely eliminate acute lymphoblastic leukemia cells from the body of the pediatric patients. Importantly, this treatment resulted not only in complete hematologic remission but in complete molecular remission in that minimal residual disease (MRD) positive acute lymphoblastic leukemia (ALL) has been converted into an MRD negative status. The treatment was well tolerated. In light of this, the administration of the CD19xCD3 bispecific single chain antibody described herein provides an improved treatment option for pediatric acute lymphoblastic leukemia (ALL), in particular to ALL refractory to chemotherapy and/or allogeneic HSCT and/or relapsed ALL.

The results may be briefly summarized as follows:
- Two pediatric patients have been treated on a compassionate use basis.
- Ongoing MRD negativity in said patients.
- No major adverse events (AEs) observed.
- All AEs transient, no treatment interruption necessary.

## Claims

1. A CD19xCD3 bispecific single chain antibody construct for use in a method for the treatment, amelioration or elimination of pediatric acute lymphoblastic leukemia (ALL) in a pediatric ALL patient.

2. The construct for the use of claim 1, wherein said pediatric acute lymphoblastic leukemia (ALL) is pediatric B-lineage acute lymphoblastic leukemia (ALL), preferably pediatric B-precursor acute lymphoblastic leukemia ALL, more preferably pediatric pro-B ALL, pre-B ALL or common ALL (cALL).

3. The construct for the use of claim 1 or 2, wherein said acute lymphoblastic leukemia (ALL) is refractory and/or relapsed ALL.

4. The construct for the use of any of claims 1 to 3, wherein the method is for the treatment, amelioration or elimination of minimal residual disease (MRD) in a pediatric ALL patient.

5. The construct for the use of claim 4, wherein said pediatric ALL patient is MRD-positive in complete hematological remission.

6. The construct for the use of claim 4 or 5, wherein MRD is measured with quantitative detection of at least one of the cytogenetic abnormalities or rearrangements selected from the group consisting of:
t(12;21)[TEL-AML1];
t(1;19;)[E2A-PBX];
t(4;11)[AF4-MLL];
t(9;22)[BCR-ABL];
hyperdiploidy or trisomies of chromosomes 4, 10, and 17;
hypodiploidy;
rearrangements of immunoglobulin genes; and
T-cell receptor (TCR) rearrangements.

7. The construct for the use of any one of claims 1 to 6, wherein the corresponding variable heavy chain regions (VH) and the corresponding variable light chain regions (VL) in said CD19xCD3 bispecific single chain antibody construct are arranged, from N-terminus to C-terminus, in the order, VL(CD19)-VH(CD19)-VH(CD3)-VL(CD3).

8. The construct for the use of claim 7, wherein said CD19xCD3 bispecific single chain antibody construct comprises an amino acid sequence as set forth in SEQ ID NO. 1, or an amino acid sequence at least 90%, preferably 95% identical to SEQ ID NO. 1.

9. The construct for use of any of claims 1 to 8, wherein the pharmaceutical composition comprising a CD19xCD3 bispecific single chain antibody construct is to be administered by continuous infusion for at least four weeks followed by a 2-week treatment-free interval.

10. The construct for the use of claim 9, wherein said administration is to be repeated at least two, three, four, five, six, seven, eight, nine orten times, after determination of a MRD negative status.

11. The construct for the use of claim 9 or 10, wherein the method is prior to allogeneic stem cell transplantation (HSCT) to convert the MRD positive ALL into an MRD negative status.

12. The construct for the use of claim 9 or 10, wherein the method is after allogeneic hematopoietic stem cell transplantation (HSCT).

13. The construct for the use of claim 12, wherein a CD19xCD3 bispecific single chain antibody construct induces a graft-versus-leukemia (GvL) effect.

14. The construct for the use of any one of claims 1 to 13, wherein the CD19xCD3 bispecific single chain antibody construct is to be administered in a daily dose of 10µg to 100µg per square meter patient body surface area.

15. The construct for the use of any one of claims 1 to 13, wherein the CD19xCD3 bispecific single chain antibody construct is to be administered in a daily dose of 15µg to 30µg per square meter patient body surface area.

16. The construct for the use of any one of claims 1 to 13, wherein the CD19xCD3 bispecific single chain antibody construct is to be administered in a daily dose of 15µg, 30µg, 60µg, or 90µg per square meter patient body surface area.

17. The construct for the use of any one of claims 1 to 13, wherein the CD19xCD3 bispecific single chain antibody construct is to be administered in a dose of 5µg at the first day(s), followed by administration of 15µg for the following day(s), followed by administration of 30 or 45µg per square meter patient body surface area as daily dose for the remaining administration period of a total of 4 weeks.

18. The construct for the use of claim 17, wherein the two initial doses are administered for 2, 3, 4, 5, 6, or 7 days or even longer.

19. The construct for the use of claim 1 or 2, wherein said patient is non-eligible for allogeneic stem cell transplantation.

## Patentansprüche

1. CD19xCD3 bispezifisches Einzelketten-Antikörper-Konstrukt zur Verwendung in einem Verfahren zur Behandlung, Linderung oder Eliminierung von pädiatrischer akuter lymphoblastischer Leukämie (ALL) in einem pädiatrischen ALL-Patienten.

2. Konstrukt zur Verwendung von Anspruch 1, wobei die pädiatrische akute lymphoblastische Leukämie (ALL) eine pädiatrische B-Linien akute lymphoblastische Leukämie (ALL) ist, vorzugsweise eine pädiatrische B-Vorläufer akute lymphoblastische Leukämie (ALL), insbesondere eine pädiatrische Pro-B-ALL, Prä-B-ALL oder Common-ALL (cALL).

3. Konstrukt zur Verwendung nach Anspruch 1 oder 2, wobei die akute lymphatische Leukämie (ALL) eine refraktäre und/oder eine rezidivierte ALL ist.

4. Konstrukt zur Verwendung nach einem der Ansprüche 1 bis 3, wobei das Verfahren zur Behandlung, Linderung oder Eliminierung einer minimalen Resterkrankung (MRD) in einem pädiatrischen ALL-Patienten dient.

5. Konstrukt zur Verwendung nach Anspruch 4, wobei der pädiatrische ALL-Patient MRD-positiv in einer kompletten hämatologischen Remission ist.

6. Konstrukt zur Verwendung nach Anspruch 4 oder 5, wobei MRD mit quantitativem Nachweis von mindestens einer der zytogenetischen Anormalitäten oder Umlagerungen gemessen wird, ausgewählt aus der Gruppe bestehend aus:
t (12; 21) [TEL-AML1];
t (1; 19;) [E2A-PBX];
t (4; 11) [AF4-MLL];
t (9; 22) [BCR-ABL];
Hyperdiploidie oder Trisomie der Chromosomen 4, 10 und 17; Hypodiploidie; Umlagerungen von Immunglobulin-Genen; und T-Zell-Rezeptor (TCR)-Umlagerungen.

7. Konstrukt zur Verwendung nach einem der Ansprüche 1 bis 6, wobei die entsprechenden variablen Regionen der schweren Kette (VH) und die entsprechenden variablen Regionen der leichten Kette (VL) in dem CD19xCD3 bispezifischen Einzelketten-Antikörper-Konstrukt, von N-Terminus zu C-Terminus, in der Reihenfolge VL (CD 19) -VH (CD 19) -VH (CD3) VL (CD3) angeordnet sind.

8. Konstrukt zur Verwendung nach Anspruch 7, wobei das CD19xCD3 bispezifische Einzelketten-Antikörper-Konstrukt eine Aminosäuresequenz, wie in SEQ ID NO. 1 gezeigt, umfasst, oder eine Aminosäuresequenz, die zu mindestens 90%, bevorzugt 95%, mit SEQ ID NO. 1 identisch ist.

9. Konstrukt zur Verwendung nach einem der Ansprüche 1 bis 8, wobei die pharmazeutische Zusammensetzung, die ein CD19xCD3 bispezifisches Einzelketten-Antikörper-Konstrukt umfasst, durch kontinuierliche Infusion für mindestens vier Wochen, gefolgt von einem 2-wöchigen behandlungsfreien Intervall, zu verabreichen ist.

10. Konstrukt zur Verwendung nach Anspruch 9, wobei die Verabreichung wenigstens zwei, drei, vier, fünf, sechs, sieben, acht, neun oder zehn Mal zu wiederholen ist, nach der Bestimmung eines MRD negativen Status.

11. Konstrukt zur Verwendung nach Anspruch 9 oder 10, wobei das Verfahren vor einer allogenen Stammzelltransplantation (HSCT) stattfindet, um eine MRD-positive ALL in einen MRD-negativen Status zu wandeln.

12. Konstrukt zur Verwendung nach Anspruch 9 oder 10, wobei das Verfahren nach einer allogenen hämatopoetischen Stammzelltransplantation (HSCT) stattfindet.

13. Konstrukt zur Verwendung nach Anspruch 12, wobei ein CD19xCD3 bispezifische Einzelketten-Antikörper-Konstrukt einen "Graft-versus-Leukemia"- (GVL)-Effekt induziert.

14. Konstrukt zur Verwendung nach einem der Ansprüche 1 bis 13, wobei das CD19xCD3 bispezifische Einzelketten-Antikörper-Konstrukt in einer täglichen Dosis von 10 µg bis 100 µg pro Quadratmeter Körperoberfläche des Patienten zu verabreichen ist.

15. Konstrukt zur Verwendung nach einem der Ansprüche 1 bis 13, wobei das CD19xCD3 bispezifische Einzelketten-Antikörper-Konstrukt in einer täglichen Dosis von 15 µg bis 30 µg pro Quadratmeter Körperoberfläche des Patienten zu verabreichen ist.

16. Konstrukt zur Verwendung nach einem der Ansprüche 1 bis 13, wobei das CD19xCD3 bispezifische Einzelketten-Antikörper-Konstrukt in einer täglichen Dosis von 15 µg, 30 µg, 60 µg oder 90µg pro Quadratmeter Körperoberfläche des Patienten zu verabreichen ist.

17. Konstrukt zur Verwendung nach einem der Ansprüche 1 bis 13, wobei das CD19xCD3 bispezifische Einzelketten-Antikörper-Konstrukt in einer Dosis von 5 µg an dem/den ersten Tag(en) zu verabreichen ist, gefolgt von einer Verabreichung von 15 µg an dem/den darauffolgenden Tag(en), gefolgt von einer Verabreichung von 30 oder 45 µg pro Quadratmeter Körperoberfläche des Patienten als Tagesdosis für den verbleibenden Verabreichungszeitraum von insgesamt 4 Wochen.

18. Konstrukt zur Verwendung nach Anspruch 17, wobei die zwei anfänglichen Dosen für 2, 3, 4, 5, 6 oder 7 Tage oder länger verabreicht werden.

19. Konstrukt zur Verwendung nach Anspruch 1 oder 2, wobei der Patient nicht für eine allogene Stammzelltransplantation geeignet ist.

## Revendications

1. Construction d'anticorps à chaîne unique bispécifique CD19xCD3 destinée à être utilisée dans un procédé pour le traitement, l'amélioration ou l'élimination d'une leucémie aiguë lymphoblastique (LAL) pédiatrique chez un patient LAL pédiatrique.

2. Construction destinée à être utilisée selon la revendication 1, dans laquelle ladite leucémie aiguë lymphoblastique (LAL) pédiatrique est une leucémie aiguë lymphoblastique (LAL) de lignée B pédiatrique, de préférence une leucémie aiguë lymphoblastique LAL à précurseurs B pédiatrique, plus préférablement une LAL pro-B, LAL pre-B ou LAL commune (cLAL) pédiatrique.

3. Construction destinée à être utilisée selon la revendication 1 ou 2, dans laquelle ladite leucémie aiguë lymphoblastique (LAL) est une LAL réfractaire et/ou en rechute.

4. Construction destinée à être utilisée selon l'une quelconque des revendications 1 à 3, dans laquelle le procédé est pour le traitement, l'amélioration ou l'élimination de la maladie résiduelle minimale (MRD) chez un patient LAL pédiatrique.

5. Construction destinée à être utilisée selon la revendication 4, dans laquelle ledit patient LAL pédiatrique est positif à la MRD en rémission hématologique complète.

6. Construction destinée à être utilisée selon la revendication 4 ou 5, dans laquelle la MRD est mesurée avec la détection quantitative d'au moins un(e) des anomalies ou remaniements cytogénétiques sélectionné(e)s parmi le groupe constitué de :
t(12 ; 21)[TEL-AML1] ;
t(1 ; 19 ;)[E2A-PBX] ;
t(4 ; 11)[AF4-MLL] ;
t(9 ; 22)[BCR-ABL] ;
une hyperdiploïdie ou des trisomies des chromosomes 4, 10 et 17 ;
une hypodiploïdie ;
des remaniements de gènes d'immunoglobuline ; et
des remaniements de récepteurs de lymphocytes T (TCR).

7. Construction destinée à être utilisée selon l'une quelconque des revendications 1 à 6, dans laquelle les régions de chaîne lourde variable (VH) correspondantes et les régions de chaîne légère variable (VL) correspondantes dans ladite construction d'anticorps à chaîne unique bispécifique CD19xCD3 sont agencées de la terminaison N à la terminaison C, dans l'ordre, VL(CD19)-VH(CD19) - VH(CD3)-VL(CD3).

8. Construction destinée à être utilisée selon la revendication 7, dans laquelle ladite construction d'anticorps à chaîne unique bispécifique CD19xCD3 comprend une séquence d'acides aminés telle qu'établie dans SEQ ID N° 1 ou une séquence d'acides aminés au moins identique à 90 %, de préférence 95 % à SEQ ID N° 1.

9. Construction destinée à être utilisée selon l'une quelconque des revendications 1 à 8, dans laquelle la composition pharmaceutique comprenant une construction d'anticorps à chaîne unique bispécifique CD19xCD3 doit être administrée par perfusion continue pendant au moins quatre semaines suivie d'un intervalle exempt de traitement de 2 semaines.

10. Construction destinée à être utilisée selon la revendication 9, dans laquelle ladite administration doit être répétée au moins deux, trois, quatre, cinq, six, sept, huit, neuf ou dix fois après détermination d'un statut négatif à la MRD.

11. Construction destinée à être utilisée selon la revendication 9 ou 10, dans laquelle le procédé est avant la transplantation de cellules souches allogéniques (HSCT) pour convertir la LAL positive à la MRD en un statut négatif à la MRD.

12. Construction destinée à être utilisée selon la revendication 9 ou 10, dans laquelle le procédé est après la transplantation de cellules souches hématopoïétiques allogéniques (HSCT).

13. Construction destinée à être utilisée selon la revendication 12, dans laquelle une construction d'anticorps à chaîne unique bispécifique CD19xCD3 induit un effet greffon contre leucémie (GvL).

14. Construction destinée à être utilisée selon l'une quelconque des revendications 1 à 13, dans laquelle la construction d'anticorps à chaîne unique bispécifique CD19xCD3 doit être administrée à une dose quotidienne de 10 µg à 100 µg par mètre carré de surface corporelle du patient.

15. Construction destinée à être utilisée selon l'une quelconque des revendications 1 à 13, dans laquelle la construction d'anticorps à chaîne unique bispécifique CD19xCD3 doit être administrée à une dose quotidienne de 15 µg à 30 µg par mètre carré de surface corporelle du patient.

16. Construction destinée à être utilisée selon l'une quelconque des revendications 1 à 13, dans laquelle la construction d'anticorps à chaîne unique bispécifique CD19xCD3 doit être administrée à une dose quotidienne de 15 µg, 30 µg, 60 µg ou 90 µg par mètre carré de surface corporelle du patient.

17. Construction destinée à être utilisée selon l'une quelconque des revendications 1 à 13, dans laquelle la construction d'anticorps à chaîne unique bispécifique CD19xCD3 doit être administrée à une dose de 5 µg le(s) premier(s) jour(s), suivie d'une administration de 15 µg le(s) jour(s) suivant(s), suivie d'une administration de 30 ou 45 µg par mètre carré de surface corporelle du patient comme dose quotidienne pour la période d'administration restante d'un total de 4 semaines.

18. Construction destinée à être utilisée selon la revendication 17, dans laquelle les deux doses initiales sont administrées pendant 2, 3, 4, 5, 6 ou 7 jours ou même plus longtemps.

19. Construction destinée à être utilisée selon la revendication 1 ou 2, dans laquelle ledit patient n'est pas éligible à la transplantation de cellules souches allogéniques.
